# EUROPEAN PATENT SPECIFICATION

(11) **EP 1 372 661 B1**
(45) Date of publication and mention of the grant of the patent: **17.05.2006**
(21) Application number: 02721191.1
(22) Date of filing: 26.02.2002
(51) Int. Cl.: A61K 31/52, A61P 1/00, A61P 1/14, A61K 31/522

(54) **GLYCOL DERIVATVES OF XANTHINES FOR TREATING IRRITABLE BOWEL SYNDROME**
XANTHIN-GLYCOL-DERIVATE ZUR BEHANDLUNG DES REIZDARMSYNDROMS
DERIVES GLYCOLIQUES DE XANTHINES POUR LE TRAITEMENT DU SYNDROME DU COLON IRRITABLE

(30) Priority: 28.02.2001 US 272115 P
(43) Date of publication of application: 02.01.2004
(73) Proprietor: SMITHKLINE BEECHAM CORPORATION, Philadelphia, PA 19101 (US)
(72) Inventor: HUBER, Brian, E., Research Triangle Park, NC 27709 (US); MANGEL, Allen, Wayne, Chapel Hill, NC 27514 (US)
(74) Representative: Phillips, Suzanne J.
(86) International application number: PCT/US2002/005973
(87) International publication number: WO 2002/067942

(56) References cited:
- WO-A-00/09507
- WO-A-98/35966
- WO-A-99/43673
- US-A- 5 017 577

## Description

The present invention relates to the use of certain glycol derivatives of xanthines, in medicine, particularly in the treatment and prophylaxis of irritable bowel syndrome.

Irritable bowel syndrome is a disease diagnosed positively by the presence of clinical features meeting the Rome criteria and by the exclusion of organic pathology justifying the symptoms. The Rome criteria for irritable bowel syndrome include continuous or recurrent symptoms of: abdominal pain or discomfort that is releived by defaecation; and/or associated with a change in frequency of stool; and/or associated with a change in consistency of stool; and two or more of the following: altered stool frequency, altered stool form, passage of mucus, and bloating or feeling of abdominal distention. IBS symptoms are reported in up to 22% of the population, with prevalence in women.

Certain pathophysiological mechanisms are known to lead to or aggravate irritable bowel syndrome, including abnormal motility, abnormal visceral perception, psychological distress and luminal factors irritating the small bowel or colon such as lactose, bile acids, short-chain fatty acids and food allergans.

IBS may present as diahrrea-predominant, constipation-predominant or alternating diarrhea and constipation forms.

Conventional treatments for IBS are directed toward treating the symptoms of the disease. Smooth muscle relaxant medications such as mebevarine have been employed. Alosetron, a 5HT3 antagonist, was recently approved for the treatment of diahrrea-predominant IBS. There remains a need for new methods for the treatment of IBS.

PCT Publication No. WO9943673 published 2 September 1999 to Zenyaku Kogyo Kabushiki Kaisha describes 1-Azaindolizine derivatives effective in actively preventing the adhesion of white blood cells and vascular endothelial cells, as an anti-inflammatory agent, an anti-allergic agent, an antisepsis shock agent, an agent to treat autoimmune diseases, an organ transplant rejection inhibitor, an agent to treat reperfusion disorder in ischemic diseases and an anti-metastasis cancer agent.

PCT Publication No. WO 9604280 published 15 February 1996 to Glaxo Group Limited describes compounds of formula: 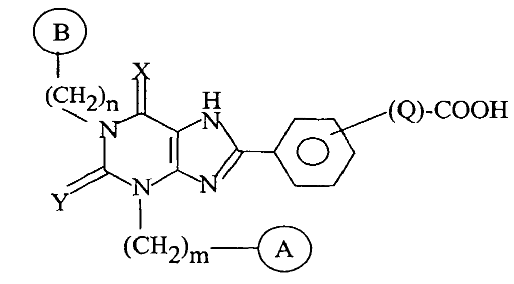 wherein m and n are independently integers from 0 to 10;
X and Y are independently oxygen or sulphur;
(-Q-) is (-CH₂-)ₚ or (-CH=CH-)ₚ where p is an integer of from 1 to 4; and
A and B are independently methyl, branched C₃₋₆ alkyl, C₃₋₈ cycloalkyl or C₃₋₈ cycloalkenyl;
and salts, solvates and pharmaceutically acceptable esters and amides thereof; and their use in treatment of inflammatory diseases, immune disorders, septic shock, circulatory disorders and gastrointestinal inflammation, infection or damage.

PCT Publication No. WO 98/35966, published 20 August 1998 to Glaxo Group Limited describes compounds of formula (I): 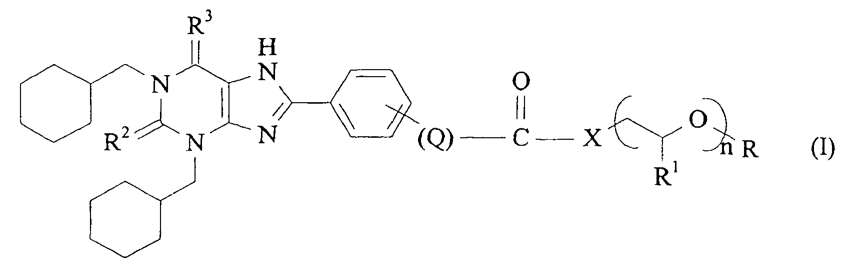 or a solvate thereof wherein:
X is -O- or -NH-;
Q is (-CH₂-)ₚ, (-CH=CH-)ₚ, (-C≡C-)ₚ where p is an integer of from 0 to 4;
R¹ is hydrogen or methyl;
R² and R³ independently represent O or S
n is an integer of 1 to 50; and
R is hydrogen or methyl;
and solvates and amides thereof; and their use in treatment of inflammatory conditions and immune disorders.

PCT Publication No. WO00/09507, published 24 February 2000 to Glaxo Group Limited describes compounds of formula (I) : 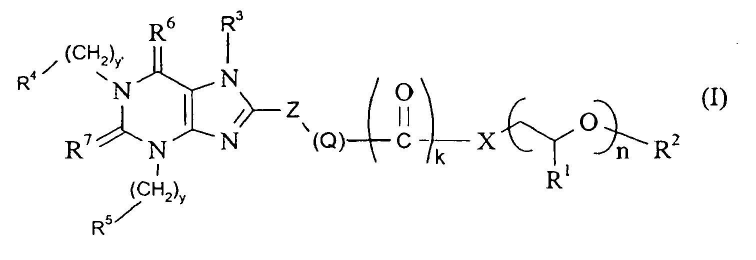 wherein
Z represents a 5 or 6 membered cycloalkyl, aryl, substituted cycloalkyl, or substituted aryl, said cycloalkyl, aryl, substituted cycloalkyl, or substituted aryl optionally containing one or more heteroatoms selected from O, N or S;
R¹ represents hydrogen or methyl;
R² represents hydrogen, C₁₋₁₂, alkyl, aryl, or aralkyl;
k represents 0 or 1
n represents an integer of 1 to 50;
X represents -O-, -N(H)-, -N(C₁₋₆alkyl)-, -N(C₃₋₈cycloalkyl)-, -N(C₁₋₈alkyl)(C₃₋₈ cycloalkyl), -N[(CH₂CH₂O)ₘ(C₁₋₁₂ alkyl, aryl, or aralkyl)]-, -CH₂O-, -CH₂NH-, -CH₂N(C₁₋₆alkyl)-, -CH₂N(C₃₋₈cycloalkyl)-, or -C₁₋₁₂alkyl-,
m represents 0-12
Q represents (-CH₂)ₚ, (-CH=CH-)p, (-C≡C-)ₚ, (-(O)ₚ₁CH2-)ₚ or (-CH₂(O)ₚ₁)ₚ where p and p¹ independently represent an integer of from 0 to 4;
y and y' independently represent integers from 0 to 10;
R³ represents H, straight or branched C₁₋₁₂alkyl (optionally substituted by phenyl, -CO- phenyl, CN, -CO(C₁₋₃) alkyl, -CO₂(C₁₋₃)alkyl, or containing one or more O atoms in the alkyl chain); C₁₋₆ straight or branched alkenyl (optionally substituted by phenyl, -CO- phenyl, CN, -CO(C₁₋₃) alkyl, -CO₂(C₁₋₃)alkyl, or containing one or more O atoms in the alkyl chain); C₁₋₆ straight or branched alkynyl or a group-C₁₋₃alkyl -NR⁸R⁹
wherein R⁸ and R⁹ are independently H, C₁₋₃alkyl or together form a 5 or 6 membered heterocyclic group, optionally containing other heteroatoms selected from O, N or S;
R⁴ and R⁵ independently represent
- C₃₋₈ cycloalkyl
- straight chain or branched C₁₋₆alkyl
- hydrogen
- straight or branched C₂₋₆alkenyl
- aryl or substituted aryl;
- heterocyclic group or subsituted heterocyclic group, including heteroaryl and substituted heteroaryl groups;
R⁶ and R⁷ independently represent O or S;
with the proviso that when
- y and y' both represent 1,
- k represents 1,
- p¹ represents zero,
- R² represents H or Me,
- R³ represents H,
- X represents O or NH, and
- Z represents phenyl
R⁴ and R⁵ do not both represent cyclohexyl;
and solvates thereof, and their use in treatment of inflammatory diseases, immune disorders, septic shock, circulatory disorders and gastrointestinal inflammation, infection or damage.

### Brief Summary of the Invention

The present invention provides the use of a compound of formula (I): 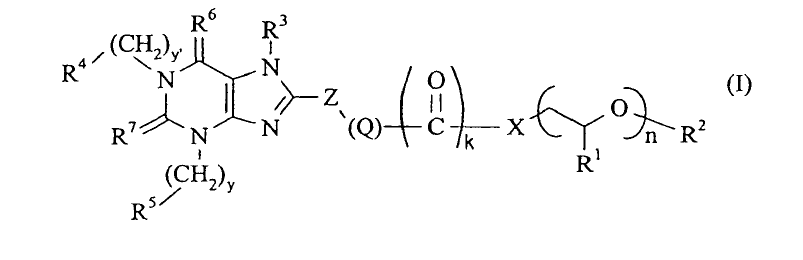 wherein:
- Z: is selected from the group consisting of C₅₋₆cycloalkyl, C₆aryl, substituted C_{5- 6}cycloalkyl, substituted C₆aryl, 5- or 6-membered heterocyclic group, substituted 5- or 6-membered heterocyclic group, 5- or 6-membered heteroaryl and substituted 5- or 6-membered heteroaryl;
- R¹: is H or methyl;
- R²: is H, C₁₋₁₂alkyl, aryl, or aralkyl;
- k: is 0 or 1;
- n: is an integer 1 to 50;
- X: is selected from the group consisting of
-O-,
-N(H)-,
-N(C₁₋₆alkyl)-,
-N(C₃₋₈cycloalkyl)-,
-N(C₁₋₈alkyl)(C₃₋₈ cycloalkyl), and
-N[(CH₂CH₂O)ₘ(C₁₋₁₂ alkyl, aryl, or aralkyl)]-;
- m: is 0-12;
- Q: is selected from the group consisting of (-CH₂)ₚ, (-CH=CH-)ₚ, (-C≡C-)ₚ, (-OCH₂-)ₚ and (-CH₂O-)ₚ where p is 0 to 4;
- y: and y' are each independently 0 to 10;
- R³: is selected from the group consisting of
H;
straight or branched C₁₋₁₂alkyl wherein said alkyl may optionally be substituted with a functional group selected from the group consisting of phenyl, -CO-phenyl, CN, -CO(C₁₋₃)alkyl, -CO₂(C₁₋₃alkyl), and wherein said C₁₋₁₂alkyl may optionally have one or more O atoms in the alkyl chain;
straight or branched C₂₋₆alkenyl;
straight or branched C₂₋₆alkynyl; and
-C₁₋₃alkyl-NR⁸R⁹ wherein R⁸ and R⁹ are each independently selected from the group consisting of H and C₁₋₃alkyl or R⁸ and R⁹ together with the N to which they are bonded form a 5- or 6-membered heterocyclic group, optionally containing 1 or 2 other heteroatoms selected from the group consisting of O, N and S;
- R⁴ and R⁵: are each independently selected from the group consisting of
- C₃₋₈cycloalkyl,
- straight or branched C₁₋₆alkyl,
- H,
- straight or branched C₂₋₆alkenyl,
- aryl,
- substituted aryl,
- heterocyclic group,
- substituted heterocyclic group,
- heteroaryl and
- substituted heteroaryl; and
- R⁶ and R⁷: are each independently O or S;
or a pharmaceutically acceptable solvate thereof, for the preparation of a medicament for the treatment or prophylaxis of irritable bowel syndrome in an animal.

### Brief Description of the Several Views of Drawings

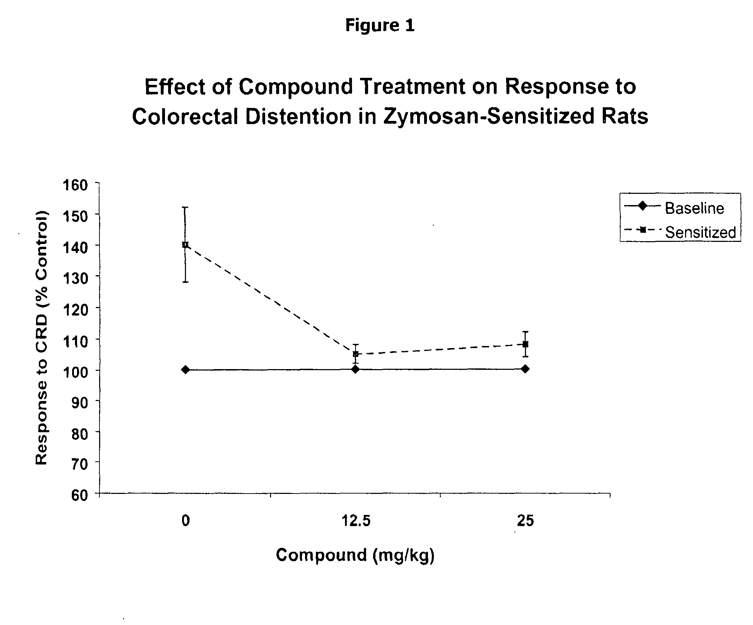

**Figure 1** is a graphical representation of the results of a study conducted in Zymosan-sensitized rats comparing the effect of (E)-4-(1,3-bis(cyclohexylmethyl)-1,2,3,6-tetrahydro-2,6-dioxo-9H-purin-8-yl)cinnamic acid nonaethylene glycol methyl ether ester (112.5 and 25 mg/kg) versus vehicle. Results are reported as response to colorectal distention (CRD) (as a percent of control) with increasing dosage of 0, 12.5, and 25 mg/kg of compound (sensitized --■--) as compared to baseline **(--◆--).**

### Detailed Description of the Invention

As used herein, the term "aryl" refers to a carbocyclic group having 6-14 carbon atoms with at least one aromatic ring (e.g., phenyl or biphenyl) or multiple condensed rings in which at least one ring is aromatic, (e.g., 1, 2, 3, 4,-tetrahydronaphthyl, naphthyl, anthryl, or phenanthryl).

As used herein, the term "substituted aryl" refers to aryl as defined above optionally substituted with one or more functional groups, e.g., halogen, lower alkyl, lower alkoxy, lower alkylthio, trifluoromethyl, amino, amido, carboxyl, hydroxyl, aryl, aryloxy, heterocycle, hetroaryl, substituted heteroaryl, nitro, cyano, alkylthio, thiol, sulfamido.

As used herein, the term "aralkyl" refers to a C₁₋₁₂alkyl that may be a straight or a branched alkyl group that is substituted by an aryl or substituted aryl group.

As used herein, the term "heterocylic group" refers to a saturated or partially unsaturated, non-aromatic group having from 5 to 12 members in a single ring (e.g. imidazolidinyl, piperidyl, piperazinyl, pyrrolidinyl, morpholinyl, pyridyl, pyridazinyl, pyrimidinyl, pyrazinyl, pyrrolyl, pyrazolyl, imidazolyl, pyranyl, furyl, thienyl, oxazolyl, isoxazolyl, oxadiazolyl, thiazylyl, thiadiazolyl, triazolyl or tetrazolyl) or multiple condensed rings (e.g. naphthpyridyl, quinoxalyl, indolizinyl or benzo[b]thienyl) and having 1, 2 or 3 heteroatoms selected from the group consisting of N, O, and S, within the ring. The heterocyclic group can optionally be unsubstituted or substituted (i.e., a "substituted heterocyclic group") with e.g. halogen, lower alkyl, lower alkoxy, lower alkylthio, trifluoromethyl, amino, amido, carboxyl, hydroxyl, aryl, aryloxy, heterocyclic group, hetroaryl, substituted heteroaryl, nitro, cyano, alkylthio, thiol, sulfamido.

As used herein, the term "heteroaryl" refers to a heterocyclic group as defined above in which at least one ring is aromatic.

As used herein, the term "substituted heteroaryl" refers to a heterocyclic group optionally substituted with one or more substituents including halogen, lower alkyl, lower alkoxy, lower alkylthio, trifluoromethyl, amino, amido, carboxyl, hydroxyl, aryl, aryloxy, heterocycle, hetroaryl, substituted heteroaryl, nitro, cyano, alkylthio, thiol, sulfamido.

The term "membered" in reference to any of cycloalkyl, substituted cycloalkyl, aryl, substituted aryl, heterocyclic group, substituted heterocyclic group, heteroaryl and substituted heteroaryl refers to the total number of atoms (C, N, O and S) in the ring. Thus a 6-membered aryl is phenyl and a 6-membered heteroaryl is pyridine.

The term "alkyl" as used herein represents straight or branched hydrocarbon chains containing the indicated number of carbon atoms.

The term "alkenyl" refers to straight or branched hydrocarbon chains containing the specified number of carbon atoms and one or more double bonds, for example propenylene.

The term "cycloalkyl" includes non-aromatic carbocyclic groups containing the specified number of carbon atoms and one or more double bonds, such as cyclopropane, cyclobutane, cyclopentane, cyclohexane, cycloheptane and cyclooctane and includes bridged cycloalkyl groups, for example norbornyl.

As used herein, the terms "substituted alkyl" and "substituted cycloalkyl" refer to alkyl and cycloalkyl optionally substituted with one or more functional groups, e.g., halogen, lower alkyl, lower alkoxy, lower alkylthio, trifluoromethyl, amino, amido, carboxyl, hydroxyl, aryl, aryloxy, heterocycle, hetroaryl, substituted heteroaryl, nitro, cyano, alkylthio, thiol, sulfamido

The term "pharmaceutically acceptable solvate" as used herein refers to a complex of variable stoichiometry formed by a solute (a compound of formula (I)) and a solvent. Solvents, by way of example, include water, methanol, ethanol, or acetic acid.

In one particular aspect, the invention provides a compound of formula (I) wherein R⁴ and R⁵ are each independently selected from the group consisting of:
- C₃₋₈cycloalkyl;
- straight or branched C₁₋₆alkyl;
- H; and
- straight or branched C₂₋₆alkenyl.

In one embodiment, the compound of formula (I) is defined where R⁴ and R⁵ are each independently aryl or substituted aryl.

In another embodiment, the compound of formula (I) is defined where R⁴ and R⁵ are each independently selected from the group consisting of a heterocyclic group, substituted heterocyclic group, heteroaryl and substituted heteroaryl groups.

In another embodiment, the compound of formula (I) is defined where R³ is H or C₁₋₃alkylNR⁸R⁹ and R⁸ and R⁹ are each independently H or C₁₋₃alkyl. In another embodiment, the compound of formula (I) is defined where R³ is C₁₋₃alkylNR⁸R⁹ and R⁸ and R⁹ together with the N to which they are bonded form a 5 or 6 membered heterocyclic group, optionally containing 1 or 2 other heteroatoms selected from the group consisting of O, N and S.

In another embodiment, the compound of formula (I) is defined where Z is selected from the group consisting of a C₅₋₆cycloalkyl, C₆aryl, substituted C₅₋₆cycloalkyl and substituted C₆aryl.

In another embodiment, the compound of formula (I) is defined where Z is selected from the group consisting of a 5- or 6-membered heterocyclic group, substituted heterocyclic group, heteroaryl and substituted heteroaryl containing from one to three heteroatoms independently selected from O, N or S.

In one preferred embodiment, the compounds of formula (I) are defined where Z is a phenyl ring, thiophene ring or pyridine ring, more preferably phenyl.

The grouping 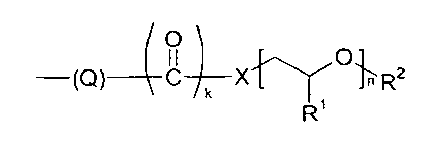 may be attached to Z in any suitable position. When Z is phenyl, preferably this group is attached to the phenyl ring in the para position.

In one preferred embodiment, the compounds of formula (I) are defined where R¹ is H or methyl.

In another preferred embodiment, the compounds of formula (I) are defined where R₂ is H, methyl or ethyl.

In one preferred embodiment, the compounds of formula (I) are defined where k is 1.

In one embodiment, the compounds of formula (I) are defined wherein n is 5-50. A preferred set of compounds of formula (I) are defined where n is from 8 to 20, more preferably from 8 to 15. However in certain embodiments of the present invention, such as wherein R³ is other than H, n may preferably be shorter than 8 to 20, such as 5 to 20. Similarly, when k is 0, n may preferably be shorter than 8 to 20, such as 5-20.

Still another preferred set of compounds of formula (I) is defined where X is -O-, -N(H)-, -N(C₁₋₆alkyl)- or -N(C₃₋₈cycloalkyl)-. More preferably, X is -O-, -N(H)- or-N(CH₃)-.

In one preferred embodiment, the compounds of formula (I) are defined where Q is
(-CH₂-)ₚ or (-CH=CH-)ₚ. In one embodiment, p is 0-2, preferably 0-1. More preferably, compounds of formula (I) are defined where Q is (-CH₂-)ₚ or (-CH=CH-)ₚ and p is 0-4, more preferably 0-2.

One preferred set of compounds of formula (I) are defined where y and y' are the same. More preferably, compounds of formula I are defined where y and y' are both 1.

In another preferred embodiment, the compounds of formula I are defined where R₃ is methyl.

Another set of preferred compounds of formula (I) are defined where R⁴ and R⁵ are each independently selected from the group consisting of C₁₋₆alkyl, C₃₋₈cycloalkyl and aryl. More preferably, R⁴ and R⁵ are each independently selected from cyclobutyl, cyclopentyl, cyclohexyl, propyl, butyl, isopropyl, isobutyl, and phenyl. Although one preferred set of compounds is defined where R⁴ and R⁵ are different, another preferred set of compounds is defined where R⁴ and R⁵ are the same.

In another preferred embodiment, R⁶ and R⁷ are the same. More preferably, both R⁶ and R⁷ are O.

According to a further aspect, the present invention provides a compound of formula (I) as defined above wherein X is -O- and R¹ is H; of these, compounds wherein n is an integer of 8 to 20 are preferred, and those wherein n is an integer of 8 to 15 are more preferred.

It is to be understood that the present invention includes all combinations and subsets of particular and preferred groups described hereinabove.

The invention also includes mixtures of compounds of formula (I) in any ratio wherein n varies.

In one embodiment, the present invention provides the use of a compound of formula (I-A): 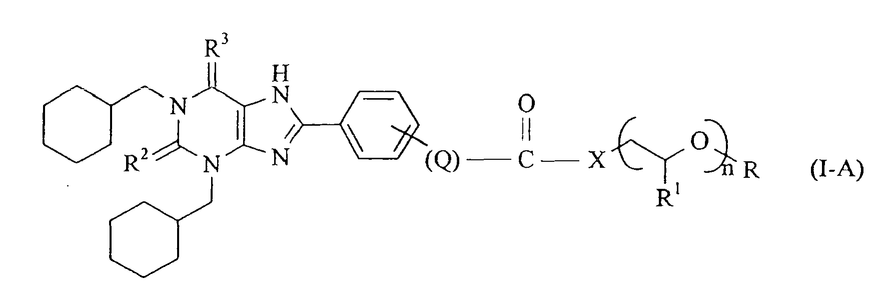 wherein:
X is -O- or -NH-;
Q is (-CH₂-)ₚ, (-CH=CH-)ₚ or (-C≡C-)ₚ where p is 0 to 4;
R¹ is H or methyl;
R² and R³ are each independently O or S;
n is an integer 1 to 50; and
R is H or methyl;
or a pharmaceutically acceptable solvate thereof, for the preparation of a medicament for the treatment or prophylaxis of irritable bowel syndrome in an animal.

Particularly preferred compounds for use in the invention include:
(E)-4-(1,3-Bis(cyclohexylmethyl)-1,2,3,6-tetrahydro-2,6-dioxo-9H-purin-8-yl)cinnamic Acid Decaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Nonaethylene Glycol Methyl Ether Ester;
(E)-3-[1,3-Bis(cyclohexylmethyl)-1,2,3,6-tetrahydro-2,6-dioxo-9H-purin-8-yl]cinnamic Acid Nonaethylene Glycol Methyl Ether Ester;
(E)-4-[1,3-Bis(cyclohexylmethyl)-1,2,3,6-tetrahydro-2,6-dioxo-9H-purin-8-yl)cinnamic acid Nonaethylene Glycol Methyl Ether Amide
(E)-4-[1,3-Bis(cyclohexylmethyl)-1,2,3,6-tetrahydro-2,6-dioxo-9H-purin-8-yl]benzoic acid Nonaethylene Glycol Methyl Ether Ester
(E)-4-(1,3-bis(benzyl)-1,2,3,6-tetrahydro-2,6-dioxo-9H-purin-8-yl)cinnamic Acid Nonaethylene Glycol Methyl Ether Ester;
(E)-4-[1,3-Bis(cyclohexylmethyl)-1,2,3,6-tetrahydro-2,6-dioxo-9H-purin-8-yl]cinnamic Acid Nonaethylene Glycol Methyl Ether Ester;
(E)-4-(1,3-bis(cyclopentylmethyl)-1,2,3,6-tetrahydro-2,6-dioxo-9H-purin-8-yl)cinnamic Acid Nonaethylene Glycol Methyl Ether Ester;
(E)-4-(1,3-bis(propyl)-1,2,3,6-tetrahydro-2,6-dioxo-9H-purin-8-yl)cinnamic Acid Nonaethylene Glycol Methyl Ether Ester;
(E)-4-(1,3-bis(cyclopropylmethyl)-1,2,3,6-tetrahydro-2,6-dioxo-9H-purin-8-yl)cinnamic Acid Nonaethylene Glycol Methyl Ether Ester;
(E)-3-((1-propyl-3-benzyl)-1,2,3,6-tetrahydro-2,6-dioxo-9H-purin-8-yl)cinnamic Acid Nonaethylene Glycol Methyl Ether Ester;
(E)-4-(1,3-bis(cycloheptylmethyl)-1,2,3,6-tetrahydro-2,6-dioxo-9H-purin-8-yl)cinnamic Acid Nonaethylene Glycol Methyl Ether Ester;
(E)-4-(1,3-bis(cyclohexylethyl)-1,2,3,6-tetrahydro-2,6-dioxo-9H-purin-8-yl)cinnamic Acid Nonaethylene Glycol Methyl Ether Ester;
(E)-4-(1,3-bis(phenyl)-1,2,3,6-tetrahydro-2,6-dioxo-9H-purin-8-yl)cinnamic Acid Nonaethylene Glycol Methyl Ether Ester;
(E)-4-(1,3-bis(2-methyl-propyl)-1,2,3,6-tetrahydro-2,6-dioxo-9H-purin-8-yl)cinnamic Acid Nonaethylene Glycol Methyl Ether Ester;
(E)-4-((1-propyl-3-cyclohexylmethyl)-1,2,3,6-tetrahydro-2,6-dioxo-9H-purin-8-yl)cinnamic Acid Nonaethylene Glycol Methyl Ether Ester;
(E)-4-(1,3-bis(bicyclo(2.2.1)hept-2-ylmethyl)-1,2,3,6-tetrahydro-2,6-dioxo-9H-purin-8-yl)cinnamic Acid Nonaethylene Glycol Methyl Ether Ester;
(E)-4-(1-cyclohexylmethyl-3-butyl)-1,2,3,6-tetrahydro-2,6-dioxo-9H-purin-8-yl)cinnamic Acid Nonaethylene Glycol Methyl Ether Ester;
(E)-4-((1-cyclohexylmethyl-3-propyl)-1,2,3,6-tetrahydro-2,6-dioxo-9H-purin-8-yl)cinnamic Acid Nonaethylene Glycol Methyl Ether Ester;
(E)-4-(1,3-bis(benzyl)-1,2,3,6-tetrahydro-2-thioxo-6-oxo-9H-purin-8-yl)cinnamic Acid Nonaethylene Glycol Methyl Ether Ester;
(E)-4-(1-methyl-3-(3-cyanobenzyl))-1,2,3,6-tetrahydro-2,6-dioxo-9H-purin-8-yl)cinnamic Acid Nonaethylene Glycol Methyl Ether Ester;
(E)-4-((1,3-bis(3-fluorobenzyl)-1,2,3,6-tetrahydro-2,6-dioxo-9H-purin-8-yl)cinnamic Acid Nonaethylene Glycol Methyl Ether Ester;
(E)-4-((1,3-bis(2-fluorobenzyl)-1,2,3,6-tetrahydro-2,6-dioxo-9H-purin-8-yl)cinnamic Acid Nonaethylene Glycol Methyl Ether Ester;
(E)-4-((1,3-bisphenethyl)-1,2,3,6-tetrahydro-2,6-dioxo-9H-purin-8-yl)cinnamic Acid Nonaethylene Glycol Methyl Ether Ester;
(E)-4-((1-cyclohexylmethyl-3-methyl)-1,2,3,6-tetrahydro-2,6-dioxo-9H-purin-8-yl)cinnamic Acid Nonaethylene Glycol Methyl Ether Ester;
(E)-4-((1-H-3-(2-methyl-propyl)-1,2,3,6-tetrahydro-2,6-dioxo-9H-purin-8-yl)cinnamic Acid Nonaethylene Glycol Methyl Ether Ester;
(E)-4-(1,3-bis(4-fluorobenzyl)-1,2,3,6-tetrahydro-2,6-dioxo-9H-purin-8-yl)cinnamic Acid Nonaethylene Glycol Methyl Ether Ester;
(E)-4-(1,3-bis(cyclohexylmethyl)-1,2,3,6-tetrahydro-2,6-dioxo-9H-purin-8-yl)cinnamic Acid Hexaethylene Glycol dodecyl Ether Ester;
(E)-4-(1,3-bis(cyclobutylmethyl)-1,2,3,6-tetrahydro-2,6-dioxo-9H-purin-8-yl)cinnamic Acid Nonaethylene Glycol Methyl Ether Ester;
(E)-4-(1-methyl-3-cyclohexylmethyl)-1,2,3,6-tetrahydro-2,6-dioxo-9H-purin-8-yl)cinnamic Acid Nonaethylene Glycol Methyl Ether Ester;
(E)-4-(1-methyl-3-isobutyl)-1,2,3,6-tetrahydro-2,6-dioxo-9H-purin-8-yl)cinnamic Acid Nonaethylene Glycol Methyl Ether Ester;
4-(1,3-bis(cyclohexylmethyl)-1,2,3,6-tetrahydro-2,6-dioxo-9H-purin-8-yl)benzoic Acid Nonaethylene Glycol Methyl Ether Ester;
(E)-4-(1,3-bis(cyclohexyl)-1,2,3,6-tetrahydro-2,6-dioxo-9H-purin-8-yl)cinnamic Acid Nonaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Nonaethylene Glycol Methyl Ether Amide;
(E)-4-(1,3-bis(cyclopentylmethyl)-1,2,3,6-tetrahydro-6-oxo-2-thioxo-9H-purin-8-yl)cinnamic Acid Nonaethylene Glycol Methyl Ether Amide;
(E)-4-(1,3-bis(2-methyl-propyl)-1,2,3,6-tetrahydro-2,6-dioxo-9H-purin-8-yl)cinnamic Acid Nonaethylene Glycol Methyl Ether Amide;
(E)-4-((1-cyclohexylmethyl-3-propyl)-1,2,3,6-tetrahydro-2,6-dioxo-9H-purin-8-yl)cinnamic Acid Nonaethylene Glycol Methyl Ether Amide;
4-(1,3-Bis(cyclohexylmethyl)-1,2,3,6-tetrahydro-2,6-dioxo-9H-purin-8-yl)benzoic Acid Nonaethylene Glycol Methyl Ether Amide;
4-(1,3-Bis(cyclohexylmethyl)-1,2,3,6-tetrahydro-2,6-dioxo-9H-purin-8-yl)benzoic Acid-N-methyl-Nonaethylene Glycol Methyl Ether Amide;
(E)-4-(1,3-Bis(cyclohexylmethyl)-2,3,6,7-tetrahydro-2,6-dioxo-7-benzyl-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-oxo-2-phenylethyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-methyl-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-propynyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo7-(2-oxo-2-methylethyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(3-morpholinopropyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-ethyl-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-ethoxy-2-oxoethyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-methyl-2-propenyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(cyanomethyl)-1H-purin-8-yl)cinnamic Acid Nonaethylene Glycol Methyl Ether Ester;
4-(1,3-Bis(cyclohexylmethyl)-2,3,6,7-tetrahydro-2,6-dioxo-7-benzyl-1H-purin-8-yl)benzoic Acid Nonaethylene Glycol Methyl Ether Ester;
4-(1,3-Bis(cyclohexylmethyl)-2,3,6,7-tetrahydro-2,6-dioxo-7-methyl-1H-purin-8-yl)benzoic Acid Nonaethylene Glycol Methyl Ether Ester;
4-[(1,3-Bis(cyclohexylmethyl)-2,3,6,7-tetrahydro-2,6-dioxo-7-methyl-1H-purin-8-yl)phenyl] propionic Acid Nonaethylene Glycol Methyl Ether Ester;
(E)-4-(1,3-Bis(cyclohexylmethyl)-2,3,6,7-tetrahydro-2,6-dioxo-7-methyl-1H-purin-8-yl)cinnamic Acid Nonaethylene Glycol Methyl Ether Amide;
(E)-4-(1,3-Bis(cyclohexylmethyl)-2,3,6,7-tetrahydro-2,6-dioxo-7-benzyl-1H-purin-8-yl)cinnamic Acid Nonaethylene Glycol Methyl Ether Amide;
4-(1,3-Bis(cyclohexylmethyl)-2,3,6,7-tetrahydro-2,6-dioxo-7-benzyl-1H-purin-8-yl)benzoic Acid Nonaethylene Glycol Methyl Ether Amide;
4-(1,3-Bis(cyclohexylmethyl)-2,3,6,7-tetrahydro-2,6-dioxo-7-methyl-1H-purin-8-yl)benzoic Acid Nonaethylene Glycol Methyl Ether Amide;
1,3-Bis(cyclohexylmethyl)-8-[4-(2,5,8,11,14,17,20,23,26,29-decaoxatriacont-1-yl)phenyl]-3,7-dihydro-1H-purine-2,6-dione;
(E)-3-[5-[1,3-bis(cyclohexylmethyl)-2,3,6,7-tetrahydro-2,6-dioxo-1H-purin-8-yl]-2-thienyl]-2-propenoic Acid Nonaethylene Glycol Methyl Ether Ester;
6-(1,3-Bis(cyclohexylmethyl)-1,2,3,6-tetrahydro-2,6-dioxo-9H-purin-8-yl)nicotinic Acid Nonaethylene Glycol Methyl Ether Amide;
(E)-3-(1,3-Bis(cyclohexylmethyl)-1,2,3,6-tetrahydro-2,6-dioxo-9H-purin-8-yl)cinnamic Acid N-cyclopropylmethyl Nonaethylene Glycol Methyl Ether Amide ;
(E)-4-(1,3-Bis(cyclohexylmethyl)-1,2,3,6-tetrahydro-2,6-dioxo-9H-purin-8-yl)cinnamic Acid Hexaethylene Glycol Benzyl Ether Amide;
(E)-4-[(3-Cyclohexylmethyl)-2,3,6,7-tetrahydro-2,6-dioxo-7-methyl-1H-purin-8-yl]cinnamic Acid Heptaethylene Glycol Methyl Ether Ester;
(E)-4-[(3-Cyclohexylmethyl)-2,3,6,7-tetrahydro-2,6-dioxo-7-methyl-1H-purin-8-yl]cinnamic Acid Nonaethylene Glycol Methyl Ether Ester;
(E)-4-[(3-Cyclohexylmethyl)-2,3,6,7-tetrahydro-2,6-dioxo-1,7-dimethyl-1H-purin-8-yl]cinnamic Acid Nonaethylene Glycol Methyl Ether Ester;
4-[1,3-Bis(cyclohexylmethyl)-1,2,3,6-tetrahydro-2,6-dioxo-9H-purin-8-yl]benzylamine Heptaethylene Glycol Methyl Ether;
4-[1,3-Bis(cyclohexylmethyl)-1,2,3,6-tetrahydro-2,6-dioxo-9H-purin-8-yl]benzylamine N-Heptaethylene Glycol Methyl Ether Hydrochloride;
4-[1,3-Bis(cyclohexylmethyl)-1,2,3,6-tetrahydro-2,6-dioxo-9H-purin-8-yl]benzylamine N-Nonaethylene Glycol Methyl Ether;
1,3-Bis(cyclohexylmethyl)-8-[3-(2,5,8,11,14,17,20,23,26,29-decaoxatriacont-1-yl)phenyl]-3,7-dihydro-1H-purine-2,6-dione;
(E)-4-(1,3-Bis(cyclohexylmethyl)-2,3,6,7-tetrahydro-2,6-dioxo-7-methyl-1H-purin-8-yl)cinnamic Acid Heptaethylene Glycol Methyl Ether Ester;
(E)-4-(1,3-Bis(cyclohexylmethyl)-2,3,6,7-tetrahydro-2,6-dioxo-7-methyl-1H-purin-8-yl)cinnamic Acid Pentaethylene Glycol Methyl Ether Ester;
(E)-4-(1,3-Bis(cyclohexylmethyl)-2,3,6,7-tetrahydro-2,6-dioxo-7-propyl-1H-purin-8-yl)cinnamic Acid Nonaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Decaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Nonaethylene Glycol Methyl Ether Ester;
(E)-3-[1,3-Bis(cyclohexylmethyl)-1,2,3,6-tetrahydro-2,6-dioxo-9H-purin-8-yl]cinnamic Acid Nonaethylene Glycol Methyl Ether Ester;
(E)-4-[1,3-Bis(cyclohexylmethyl)-1,2,3,6-tetrahydro-2,6-dioxo-9H-purin-8-yl)cinnamic acid Nonaethylene Glycol Methyl Ether Amide; and
(E)-4-[1,3-Bis(cyclohexylmethyl)-1,2,3,6-tetrahydro-2,6-dioxo-9H-purin-8-yl]benzoic acid Nonaethylene Glycol Methyl Ether Ester; and
pharmaceutically acceptable solvates thereof.

More particularly preferred compounds for use in the present invention include:
(E)-4-(1,3-Bis(cyclohexylmethyl)-1,2,3,6-tetrahydro-2,6-dioxo-9H-purin-8-yl)cinnamic Acid Decaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Nonaethylene Glycol Methyl Ether Ester;
(E)-3-[1,3-Bis(cyclohexylmethyl)-1,2,3,6-tetrahydro-2,6-dioxo-9H-purin-8-yl]cinnamic Acid Nonaethylene Glycol Methyl Ether Ester;
(E)-4-[1,3-Bis(cyclohexylmethyl)-1,2,3,6-tetrahydro-2,6-dioxo-9H-purin-8-yl)cinnamic acid Nonaethylene Glycol Methyl Ether Amide;
(E)-4-[1,3-Bis(cyclohexytmethyl)-1,2,3,6-tetrahydro-2,6-dioxo-9H-purin-8-yl]benzoic acid Nonaethylene Glycol Methyl Ether Ester;
(E)-4-(1,3-bis(benzyl)-1,2,3,6-tetrahydro-2,6-dioxo-9H-purin-8-yl)cinnamic Acid Nonaethylene Glycol Methyl Ether Ester;
(E)-4-[1,3-Bis(cyclohexylmethyl)-1,2,3,6-tetrahydro-2,6-dioxo-9H-purin-8-yl]cinnamic Acid Nonaethylene Glycol Methyl Ether Ester;
(E)-4-(1,3-bis(cyclopentylmethyl)-1,2,3,6-tetrahydro-2,6-dioxo-9H-purin-8-yl)cinnamic Acid Nonaethylene Glycol Methyl Ether Ester;
(E)-4-(1,3-bis(propyl)-1,2,3,6-tetrahydro-2,6-dioxo-9H-purin-8-yl)cinnamic Acid Nonaethylene Glycol Methyl Ether Ester;
(E)-4-(1,3-bis(cycloheptylmethyl)-1,2,3,6-tetrahydro-2,6-dioxo-9H-purin-8-yl)cinnamic Acid Nonaethylene Glycol Methyl Ether Ester;
(E)-4-(1,3-bis(cyclohexylethyl)-1,2,3,6-tetrahydro-2,6-dioxo-9H-purin-8-yl)cinnamic Acid Nonaethylene Glycol Methyl Ether Ester;
(E)-4-(1,3-bis(phenyl)-1,2,3,6-tetrahydro-2,6-dioxo-9H-purin-8-yl)cinnamic Acid Nonaethylene Glycol Methyl Ether Ester;
(E)-4-(1,3-bis(2-methyl-propyl)-1,2,3,6-tetrahydro-2,6-dioxo-9H-purin-8-yl)cinnamic Acid Nonaethylene Glycol Methyl Ether Ester;
(E)-4-((1-propyl-3-cyclohexylmethyl)-1,2,3,6-tetrahydro-2,6-dioxo-9H-purin-8-yl)cinnamic Acid Nonaethylene Glycol Methyl Ether Ester;
(E)-4-(1,3-bis(bicyclo(2.2.1)hept-2-ylmethyl)-1,2,3,6-tetrahydro-2,6-dioxo-9H-purin-8-yl)cinnamic Acid Nonaethylene Glycol Methyl Ether Ester;
(E)-4-(1-cyclohexylmethyl-3-butyl)-1,2,3,6-tetrahydro-2,6-dioxo-9H-purin-8-yl)cinnamic Acid Nonaethylene Glycol Methyl Ether Ester;
(E)-4-((1-cyclohexylmethyl-3-propyl)-1,2,3,6-tetrahydro-2,6-dioxo-9H-purin-8-yl)cinnamic Acid Nonaethylene Glycol Methyl Ether Ester;
(E)-4-((1,3-bis(3-fluorobenzyl)-1,2,3,6-tetrahydro-2,6-dioxo-9H-purin-8-yl)cinnamic Acid Nonaethylene Glycol Methyl Ether Ester;
(E)-4-((1,3-bis(2-fluorobenzyl)-1,2,3,6-tetrahydro-2,6-dioxo-9H-purin-8-yl)cinnamic Acid Nonaethylene Glycol Methyl Ether Ester;
(E)-4-((1,3-bisphenethyl)-1,2,3,6-tetrahydro-2,6-dioxo-9H-purin-8-yl)cinnamic Acid Nonaethylene Glycol Methyl Ether Ester;
(E)-4-((1-H-3-(2-methyl-propyl)-1,2,3,6-tetrahydro-2,6-dioxo-9H-purin-8-yl)cinnamic Acid Nonaethylene Glycol Methyl Ether Ester;
(E)-4-(1,3-bis(4-fluorobenzyl)-1,2,3,6-tetrahydro-2,6-dioxo-9H-purin-8-yl)cinnamic Acid Nonaethylene Glycol Methyl Ether Ester;
(E)-4-(1,3-bis(cyclohexylmethyl)-1,2,3,6-tetrahydro-2,6-dioxo-9H-purin-8-yl)cinnamic Acid Hexaethylene Glycol dodecyl Ether Ester;
(E)-4-(1,3-bis(cyclobutylmethyl)-1,2,3,6-tetrahydro-2,6-dioxo-9H-purin-8-yl)cinnamic Acid Nonaethylene Glycol Methyl Ether Ester;
(E)-4-(1-methyl-3-isobutyl)-1,2,3,6-tetrahydro-2,6-dioxo-9H-purin-8-yl)cinnamic Acid Nonaethylene Glycol Methyl Ether Ester;
4-(1,3-bis(cyclohexylmethyl)-1,2,3,6-tetrahydro-2,6-dioxo-9H-purin-8-yl)benzoic Acid Nonaethylene Glycol Methyl Ether Ester;
(E)-3-(1,3-bis(cyclohexylmethyl)-1,2,3,6-tetrahydro-2,6-dioxo-9H-purin-8-yl)cinnamic Acid Nonaethylene Glycol Methyl Ether Amide;
(E)-4-(1,3-Bis(cyclohexylmethyl)-1,2,3,6-tetrahydro-2,6-dioxo-9H-purin-8-yl)cinnamic Acid-N-methyl Nonaethylene Glycol Methyl Ether Amide;
(E)-4-(1,3-Bis(cyclohexylmethyl)-1,2,3,6-tetrahydro-2,6-dioxo-9H-purin-8-yl)cinnamic Acid Nonaethylene Glycol Methyl Ether Amide;
4-(1,3-Bis(cyclohexylmethyl)-1,2,3,6-tetrahydro-2,6-dioxo-9H-purin-8-yl)benzoic Acid Nonaethylene Glycol Methyl Ether Amide;
(E)-4-(1,3-Bis(cyclohexylmethyl)-2,3,6,7-tetrahydro-2,6-dioxo-7-benzyl-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-oxo-2-phenylethyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-methyl-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-propynyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo7-(2-oxo-2-methylethyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(3-morpholinopropyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-ethyl-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-ethoxy-2-oxoethyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-methyl-2-propenyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(cyanomethyl)-1H-purin-8-yl)cinnamic Acid Nonaethylene Glycol Methyl Ether Ester;
4-(1,3-Bis(cyclohexylmethyl)-2,3,6,7-tetrahydro-2,6-dioxo-7-benzyl-1H-purin-8-yl)benzoic Acid Nonaethylene Glycol Methyl Ether Ester;
4-(1,3-Bis(cyclohexylmethyl)-2,3,6,7-tetrahydro-2,6-dioxo-7-methyl-1H-purin-8-yl)benzoic Acid Nonaethylene Glycol Methyl Ether Ester;
(E)-4-(1,3-Bis(cyclohexylmethyl)-2,3,6,7-tetrahydro-2,6-dioxo-7-methyl-1H-purin-8-yl)cinnamic Acid Nonaethylene Glycol Methyl Ether Amide;
(E)-4-(1,3-Bis(cyclohexylmethyl)-2,3,6,7-tetrahydro-2,6-dioxo-7-benzyl-1H-purin-8-yl)cinnamic Acid Nonaethylene Glycol Methyl Ether Amide;
4-(1,3-Bis(cyclohexylmethyl)-2,3,6,7-tetrahydro-2,6-dioxo-7-benzyl-1H-purin-8-yl)benzoic Acid Nonaethylene Glycol Methyl Ether Amide;
1,3-Bis(cyclohexylmethyl)-8-[4-(2,5,8,11,14,17,20,23,26,29-decaoxatriacont-1-yl)phenyl]-3,7-dihydro-1H-purine-2,6-dione;
(E)-3-[5-[1,3-bis(cyclohexylmethyl)-2,3,6,7-tetrahydro-2,6-dioxo-1H-purin-8-yl]-2-thienyl]-2-propenoic Acid Nonaethylene Glycol Methyl Ether Ester;
6-(1,3-Bis(cyclohexylmethyl)-1,2,3,6-tetrahydro-2,6-dioxo-9H-purin-8-yl)nicotinic Acid Nonaethylene Glycol Methyl Ether Amide;
(E)-3-(1,3-Bis(cyclohexylmethyl)-1,2,3,6-tetrahydro-2,6-dioxo-9H-purin-8-yl)cinnamic Acid N-cyclopropylmethyl Nonaethylene Glycol Methyl Ether Amide ;
(E)-4-[(3-Cyciohexylmethyl)-2,3,6,7-tetrahydro-2,6-dioxo-7-methyl-1H-purin-8-yl]cinnamic Acid Nonaethylene Glycol Methyl Ether Ester;
4-[1,3-Bis(cyclohexyimethyl)-1,2,3,6-tetrahydro-2,6-dioxo-9H-purin-8-yl]benzylamine N-Heptaethylene Glycol Methyl Ether Hydrochloride;
1,3-Bis(cyclohexylmethyl)-8-[3-(2,5,8,11,14,17,20,23,26,29-decaoxatriacont-1-yl)phenyl]-3,7-dihydro-1H-purine-2,6-dione;
(E)-4-(1,3-bis(cyclopentylmethyl)-1,2,3,6-tetrahydro-6-oxo-2-thioxo-9H-purin-8-yl)cinnamic Acid Nonaethylene Glycol Methyl Ether Amide;
(E)-4-(1,3-Bis(cyclohexylmethyl)-2,3,6,7-tetrahydro-2,6-dioxo-7-propyl-1H-purin-8-yl)cinnamic Acid Nonaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Decaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Nonaethylene Glycol Methyl Ether Ester;
(E)-3-[1,3-Bis(cyclohexylmethyl)-1,2,3,6-tetrahydro-2,6-dioxo-9H-purin-8-yl]cinnamic Acid Nonaethylene Glycol Methyl Ether Ester;
(E)-4-[1,3-Bis(cyclohexylmethyl)-1,2,3,6-tetrahydro-2,6-dioxo-9H-purin-8-yl)cinnamic acid Nonaethylene Glycol Methyl Ether Amide; and
(E)-4-[1,3-Bis(cyclohexylmethyl)-1,2,3,6-tetrahydro-2,6-dioxo-9H-purin-8-yl]benzoic acid Nonaethylene Glycol Methyl Ether Ester; and
pharmaceutically acceptable solvates thereof.

In one preferred embodiment, the present invention provides the use of (E)-4-(1,3-Bis(cyclohexylmethyl)-1,2,3,6-tetrahydro-2,6-dioxo-9H-purin-8-yl)cinnamic acid nonaethylene glycol methyl ether ester or a pharmaceutically acceptable solvate thereof, for the preparation of a medicament for the treatment or prophylaxis of irritable bowel syndrome.

The compounds employed in the present invention are capable of existing as geometric and optical isomers. All such isomers, individually and as mixtures, are included within the scope of the present invention. Where Q contains a double bond, compounds in the form of the E-geometric isomers are preferred.

Hereinafter reference to "compounds of formula (I)" shall include all compounds of formula (I), and specifically includes all compounds of formula (I-A), and pharmaceutically acceptable solvates thereof.

The compounds employed in the present invention are cell adhesion molecule inhibitors, and preferably endothelial cell adhesion molecule inhibitors. The term "cell adhesion molecule inhibitor" includes compounds which specifically block or inhibit proteins on the surface of animal cells that mediate cell-cell binding. Preferably, the term "cell adhesion molecule inhibitor" includes compounds which inhibit the expression of cell adhesion molecules.

The term "endothelial cell adhesion molecule inhibitor" includes compounds which specifically block or inhibit the adhesive interactions of leukocytes and the endothelium. These compounds can be identified by performing the endothelial cell adhesion assay as decribed herein below. Preferably, the compounds have IC₅₀ values in this assay of 500nM or less, more preferably 100nM or less and even more preferably 50nM or less. Preferably, the term "endothelial cell adhesion molecule inhibitor" includes compounds which inhibit the expression of endothelial cell adhesion molecules. More preferably, the endothelial cell adhesion molecules include ICAM-1 (Intercellular adhesion molecule-1), E-selectin, VCAM-1 and MadCAM.

The present invention involves treating or preventing irritable bowel syndrome, including diarrhea-predominant, constipation-predominant and alternating irritable bowel syndrome, by administering to an animal, a therapeutically effect amount of an endothelial cell adhesion molecule inhibitor, such as a compound of formula (I) or a solvate thereof. The present invention may be employed for the treatment or prophylaxis of irritable bowel syndrome in animals generally, and particularly in mammals such as humans.

The term "therapeutically effective amount" refers to an amount of an endothelial cell adhesion molecule inhibitor, e.g., a compound of formula (I), which is effective for the treatment or prophylaxis of the stated condition. Thus, a therapeutically effective amount of a compound of formula (I) for the treatment or prophylaxis of irritable bowel syndrome is an amount effective for the treatment or prophylaxis of irritable bowel syndrome. The term "treatment" as used herein refers to the partial or total elimination of symptoms in an afflicted animal. The term "prophylaxis as used herein refers to the complete prevention of the condition in an animal as well as the reduction in severity and/or frequency of symptoms of the condition in an afflicted animal.

The amount of an endothelial cell adhesion molecule inhibitor, e.g., a compound of formula (I) or pharmaceutically acceptable solvate thereof, which is required to achieve the desired biological effect will depend on a number of factors such as the use for which it is intended, the means of administration, and the recipient, and will ultimately be in the discretion of the attendent physician. A typical daily dose for the treatment of irritable bowel syndrome, for instance, may be expected to lie in the range of 0.005 mg/kg - 100mg/kg, preferably 0.5-100 mg/kg, and most preferably 0.5-20 mg/kg. This dose may be administered as a single unit dose, as several separate unit doses or as a continuous infusion.

An intravenous dose may be expected to lie in the range of 0.0025 mg/kg to 200 mg/kg and would typically be administered as an infusion.

According to the present invention, it is possible to administer the compounds of formula (I) neat, although it is preferred to administer the compounds of formula (I) in the form of a pharmaceutical formulation. Thus, in a further aspect of the present invention, there are provided pharmaceutical compositions comprising, as active ingredient, a compound of formula (I) or a pharmaceutically acceptable solvate thereof, together with at least one pharmaceutically acceptable carrier or excipient. These pharmaceutical compositions may be used in the prophylaxis or treatment of irritable bowel syndrome. The carrier must be pharmaceutically acceptable to the recipient and must be compatible with, i.e. not have a deleterious effect upon, the other ingredients in the composition. The carrier may be a solid or liquid and the formulation is preferably formulated as a unit dose formulation, for example, a tablet which may contain from 0.05 to 95% by weight of the active ingredients. If desired other physiologically active ingredients may also be incorporated in the pharmaceutical compositions of the invention. In one embodiment, the present invention comprises administering a therapeutically effective amount of a combination of a compound of formula (I) or a pharmaceutically acceptable solvate thereof and alosetron or a pharmaceutically acceptable salt thereof.
Possible formulations include those suitable for oral, sublingual, buccal, parenteral (for example subcutaneous, intramuscular, or intravenous), rectal, topical including transdermal, intranasal and inhalation administration. Most suitable means of administration for a particular patient will depend on the nature and severity of the condition being treated and on the nature of the active compound.

Formulations suitable for oral administration may be provided as discrete units, such as tablets, capsules, cachets, lozenges, each containing a predetermined amount of the active compound; as powders or granules; as solutions or suspensions in aqueous or non-aqueous liquids; or as oil-in-water or water-in-oil emulsions.

Formulations suitable for sublingual or buccal administration include lozenges comprising the active compound and, typically a flavoured base, such as sugar and acacia or tragacanth and pastilles comprising the active compound in an inert base, such as gelatine and glycerine or sucrose acacia.

Formulations suitable for parenteral administration typically comprise sterile aqueous solutions containing a predetermined concentration of the active compound; the solution is preferably isotonic with the blood of the intended recipient. Additional formulations suitable for parenteral administration include formulations containing physiologically suitable co-solvents and/or complexing agents such as surfactants and cyclodextrins. Oil-in-water emulsions are also suitable formulations for parenteral formulations. Although such solutions are preferably administered intravenously, they may also be administered by subcutaneous or intramuscular injection.

Formulations suitable for rectal administration are preferably provided as unit-dose suppositories comprising the active ingredient in one or more solid carriers forming the suppository base, for example, cocoa butter.

Formulations suitable for topical or intranasal application include ointments, creams, lotions, pastes, gels, sprays, aerosols and oils. Suitable carriers for such formulations include petroleum jelly, lanolin, polyethyleneglycols, alcohols, and combinations thereof. The active ingredient is typically present in such formulations at a concentration of from 0.1 to 15% w/w.

Formulations of the invention may be prepared by any suitable method, typically by uniformly and intimately admixing the active compound with liquids or finely divided solid carriers or both, in the required proportions and then, if necessary, shaping the resulting mixture into the desired shape.

For example a tablet may be prepared by compressing an intimate mixture comprising a powder or granules of the active ingredient and one or more optional ingredients, such as a binder, lubricant, inert diluent, or surface active dispersing agent, or by moulding an intimate mixture of powdered active ingredient and inert liquid diluent.

Suitable formulations for administration by inhalation include fine particle dusts or mists which may be generated by means of various types of metered dose pressurised aerosols, nebulisers, or insufflators.

Therefore, according to a further aspect of the present invention, there is provided the use of a compound of formula (I) or a pharmaceutically acceptable solvate thereof in the preparation of a medicament for the prophylaxis or treatment of irritable bowel syndrome.

Compounds of formula (I) may be prepared and formulated as described in PCT application publication Nos. WO 98.35966 and WO 00.09507.

The invention will now be described by way of illustration only, by the following examples:

### Cell Adhesion Assay

The antiadhesion activity of compounds described herein was determined using a modification of the previously described method, Jurgensen, C.H. et. al., *J*. *Immunol.* 1990, **144**: 653-661. The adhesiveness of cytokine-stimulated human umbilical vein endothelial cells was assessed by quantitating the adherence of fluorescently-labelled (calcein-AM, Molecular Probes, Eugene, OR) leukocytes to endothelial cell monolayers. Activity was determined by calculating inhibition of cytokine-stimulated adhesion minus the basal adhesion (unstimulated).

### Rodent Model of Zymosan-Induced Hyperalgesia

Protocol for Evaluation of (E)-4-(1,3-bis(cyclohexylmethyl)-1,2,3,6-tetrahydro-2,6-dioxo-9H-purin-8-yl)cinnamic acid nonaethylene glycol methyl ether ester in a Rodent Model of Zymosan-Induced Hyperalgesia

### Animals

Adult male Sprague-Dawley rats (400-425 g) housed 1-2 per cage in the animal care facility at the University of Iowa (approved by the American Association for Accreditation of Laboratory Animal Care). All experimental procedures were approved by the Institutional Animal Care and Use Committee at the University of Iowa.

### Surgical Preparation

Rats were deeply anesthetized with pentobarbital sodium (45 mg/kg) administered intraperitoneally. Electrodes were stitched into the external oblique musculature for electromyographic (EMG) recording. Electrode leads were tunneled subcutaneously and exteriorized at the nape of the neck for future access. After surgery, rats were housed separately and allowed to recuperate for at least 3 days prior to testing.

### Behavioral Testing

The descending colon and rectum were distended by pressure-controlled inflation of a 7-8-cm-long flexible latex balloon tied around a flexible tube. The balloon was lubricated , inserted into the colon via the anus, and anchored by taping the balloon catheter to the base of the tail. Noxious phasic colorectal distension (CRD, 80 mm Hg, 20 seconds) was achieved by opening a solenoid gate to a constant pressure air reservoir. Intracolonic pressure was continuously monitored by the aid of a pressure control device. Response was quantified as the visceromotor response (VMR), a contraction of the abdominal and hindlimb musculature. EMG activity produced by contraction of the external oblique musculature was quantified using Spike2 software (Cambridge Electronic Designs). Each distension trial lasted 60 seconds, and EMG activity was quantitated in 1-second bins for 20 seconds before distension (baseline), during distention, and 20 seconds after distention. The increase in total number of recorded counts during distention is defined as the response.

### Compound Testing

Stable baseline responses to CRD (80 mm Hg, 20 seconds, 4 minutes apart) was obtained in conscious, unsedated rats before any treatment, followed by oral gavage with 2 doses of (E)-4-(1,3-bis(cyclohexylmethyl)-1,2,3,6-tetrahydro-2,6-dioxo-9H-purin-8-yl)cinnamic acid nonaethylene glycol methyl ether ester (12.5 mg/kg and 25 mg/kg) (Time 0). Control animals received vehicle only. At time 16 hours, a pre-zymosan response to distention was measured, followed by second oral doses of experimental compound. The animals were then briefly anesthetized with halothane, and zymosan (1 mL, 25 mg/mL) was instilled into the colon with a gavage needle inserted to a depth of about 7-8 cm, to produce inflammation and enhance the VMR to CRD. Four hours after intracolonic treatment, responses to CRD were quantified as described above.

### Results Discussion

Hyperalgesia is an altered sensory state of increased sensitivity to pain. Visceral hyperalgesia associated with the gastrointestinal tract may arise secondary to infection or inflammation. Such altered visceral sensation, as exemplified by increased sensitivity to colorectal distention, has been observed in patients with functional bowel disorders. Coutinho, Meller, and Gebhart have shown that intracolonic instillation of zymosan, a yeast cell wall derivative which acts as an inflamogen, produces colonic inflammation and enhanced visceromotor responses to colorectal distention as a measurement of response to pain (Ref: Coutinho SV, Meller ST, Gebhart GF. Intracolonic zymosan produces visceral hyperalgesia in the rat that is mediated by spinal NMDA and non-NMDA receptors. Brain Res 1996; 736:7-15).

The results of the study are reported in **Figure 1.** Results from evaluation of (E)-4-(1,3-bis(cyclohexylmethyl)-1,2,3,6-tetrahydro-2,6-dioxo-9H-purin-8-yl)cinnamic acid nonaethylene glycol methyl ether ester in this model show that this compound was efficacious in decreasing zymosan-induced visceral hypersensitivity to colorectal distention. Both doses (12.5 and 25 mg/kg) of the compound effectively decreased the response to colorectal distention down to baseline levels. Results are expressed as percentage of control, with baseline levels at 100% of control. Increased hypersensitivity is evidenced by increases over 100% of responses to colorectal distention. Overall, these data indicate that (E)-4-(1,3-bis(cyclohexylmethyl)-1,2,3,6-tetrahydro-2,6-dioxo-9H-purin-8-yl)cinnamic acid nonaethylene glycol methyl ether ester is useful for the treatment and prophylaxis of irritable bowel syndrome.

## Claims

1. The use of a compound of formula (I) : 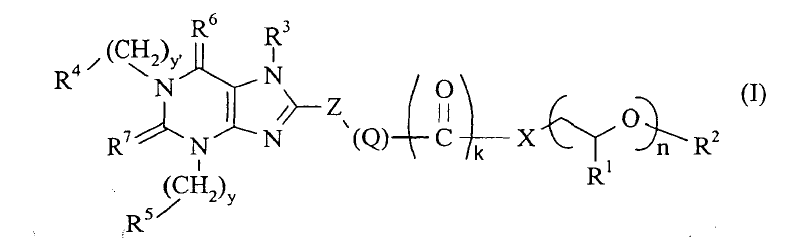 wherein:
Z is selected from the group consisting of C₅₋₆cycloalkyl, C₆aryl, substituted C₅₋₆cycloalkyl, substituted C₆aryl, 5- or 6-membered heterocyclic group, substituted 5- or 6-membered heterocyclic group, 5- or 6-membered heteroaryl and substituted 5- or 6-membered heteroaryl;
R¹ is H or methyl;
R² is H, C₁₋₁₂alkyl, aryl, or aralkyl;
k is 0 or 1;
n is an integer 1 to 50;
X is selected from the group consisting of
-O-,
-N(H)-,
-N(C₁₋₆alkyl)-,
-N(C₃₋₈cycloalkyl)-,
-N(C₁₋₈alkyl)(C₃₋₈ cycloalkyl), and
-N[(CH₂CH₂O)ₘ(C₁₋₁₂ alkyl, aryl, or aralkyl)]-;
m is 0-12;
Q is selected from the group consisting of (-CH₂)ₚ, (-CH=CH-)ₚ, (-C≡C-)ₚ, (-OCH₂-)ₚ and (-CH₂O)ₚ where p is 0 to 4;
y and y' are each independently 0 to 10;
R³ is selected from the group consisting of
H;
straight or branched C₁₋₁₂alkyl wherein said alkyl may optionally be substituted with a functional group selected from the group consisting of phenyl, -CO-phenyl, CN, -CO(C₁₋₃)alkyl, -CO₂(C₁₋₃alkyl), and wherein said C₁₋₁₂alkyl may optionally have one or more O atoms in the alkyl chain;
straight or branched C₂₋₆alkenyl;
straight or branched C₂₋₆alkynyl; and
-C₁₋₃alkyl-NR⁸R⁹ wherein R³ and R⁹ are each independently selected from the group consisting of H and C₁₋₃alkyl or R⁸ and R⁹ together with the N to which they are bonded form a 5- or 6-membered heterocyclic group, optionally containing 1 or 2 other heteroatoms selected from the group consisting of O, N and S;
R⁴ and R⁵ are each independently selected from the group consisting of
- C₃₋₈cycloalkyl,
- straight or branched C₁₋₆alkyl,
- H,
- straight or branched C₂₋₆alkenyl,
- aryl,
- substituted aryl,
- heterocyclic group,
- substituted heterocyclic group,
- heteroaryl and
- substituted heteroaryl; and
R⁶ and R⁷ are each independently O or S; where the term "aralkyl" refers to a C₁₋₁₂alkyl that may be straight or a branched alkyl group that is substituted by an aryl or substituted aryl group and the term "alkyl" represents straight or branched hydrocarbon chains containing the indicated number of carbon atoms,
or a pharmaceutically acceptable solvate thereof, for the preparation of a medicament for the treatment or prophylaxis of irritable bowel syndrome in an animal.

2. The use according to claim 1, wherein the compound of formula (I) is a compound of formula (I-A) 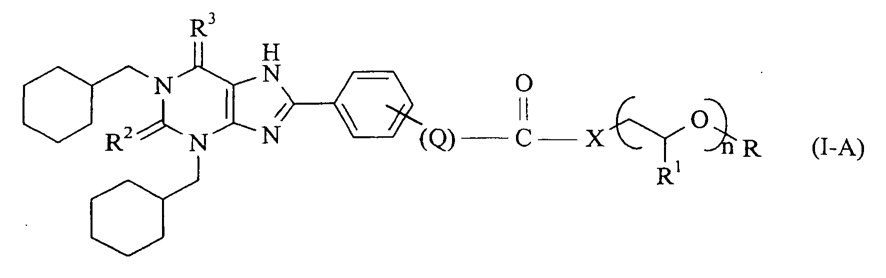 wherein:
X is -O- or -NH-;
Q is selected from the group consisting of (-CH₂-)ₚ, (-CH=CH-)ₚ and (-C≡C-)ₚ where p is 0 to 4;
R¹ is H or methyl;
R² and R³ are each independently O or S;
n is an integer 1 to 50; and
R is H or methyl;
or a pharmaceutically acceptable solvate thereof.

3. The use according to claim 1 wherein the compound of formula (I) is selected from the group consisting of:
(E)-4-(1,3-bis(benzyl)-1,2,3,6-tetrahydro-2,6-dioxo-9H-purin-8-yl)cinnamic Acid Nonaethylene Glycol Methyl Ether Ester;
(E)-4-[1,3-Bis(cyclohexylmethyl)-1,2,3,6-tetrahydro-2,6-dioxo-9H-purin-8-yl]cinnamic Acid Nonaethylene Glycol Methyl Ether Ester;
(E)-4-(1,3-bis(cyclopentylmethyl)-1,2,3,6-tetrahydro-2,6-dioxo-9H-purin-8-yl)cinnamic Acid Nonaethylene Glycol Methyl Ether Ester;
(E)-4-(1,3-bis(propyl)-1,2,3,6-tetrahydro-2,6-dioxo-9H-purin-8-yl)cinnamic Acid Nonaethylene Glycol Methyl Ether Ester;
(E)-4-(1,3-bis(cyclopropylmethyl)-1,2,3,6-tetrahydro-2,6-dioxo-9H-purin-8-yl)cinnamic Acid Nonaethylene Glycol Methyl Ether Ester;
(E)-3-((1-propyl-3-benzyl)-1,2,3,6-tetrahydro-2,6-dioxo-9H-purin-8-yl)cinnamic Acid Nonaethylene Glycol Methyl Ether Ester;
(E)-4-(1,3-bis(cycloheptylmethyl)-1,2,3,6-tetrahydro-2,6-dioxo-9H-purin-8-yl)cinnamic Acid Nonaethylene Glycol Methyl Ether Ester;
(E)-4-(1,3-bis(cyclohexylethyl)-1,2,3,6-tetrahydro-2,6-dioxo-9H-purin-8-yl)cinnamic Acid Nonaethylene Glycol Methyl Ether Ester;
(E)-4-(1,3-bis(phenyl)-1,2,3,6-tetrahydro-2,6-dioxo-9H-purin-8-yl)cinnamic Acid Nonaethylene Glycol Methyl Ether Ester;
(E)-4-(1,3-bis(2-methyl-propyl)-1,2,3,6-tetrahydro-2,6-dioxo-9H-purin-8-yl)cinnamic Acid Nonaethylene Glycol Methyl Ether Ester;
(E)-4-((1-propyl-3-cyclohexylmethyl)-1,2,3,6-tetrahydro-2,6-dioxo-9H-purin-8-yl)cinnamic Acid Nonaethylene Glycol Methyl Ether Ester;
(E)-4-(1,3-bis(bicyclo(2.2.1)hept-2-ylmethyl)-1,2,3,6-tetrahydro-2,6-dioxo-9H-purin-8-yl)cinnamic Acid Nonaethylene Glycol Methyl Ether Ester;
(E)-4-(1-cyclohexylmethyl-3-butyl)-1,2,3,6-tetrahydro-2,6-dioxo-9H-purin-8-yl)cinnamic Acid Nonaethylene Glycol Methyl Ether Ester;
(E)-4-((1-cyclohexylmethyl-3-propyl)-1,2,3,6-tetrahydro-2,6-dioxo-9H-purin-8-yl)cinnamic Acid Nonaethylene Glycol Methyl Ether Ester;
(E)-4-(1,3-bis(benzyl)-1,2,3,6-tetrahydro-2-thioxo-6-oxo-9H-purin-8-yl)cinnamic Acid Nonaethylene Glycol Methyl Ether Ester;
(E)-4-(1-methyl-3-(3-cyanobenzyl))-1,2,3,6-tetrahydro-2,6-dioxo-9H-purin-8-yl)cinnamic Acid Nonaethylene Glycol Methyl Ether Ester;
(E)-4-((1,3-bis(3-fluorobenzyl)-1,2,3,6-tetrahydro-2,6-dioxo-9H-purin-8-yl)cinnamic Acid Nonaethylene Glycol Methyl Ether Ester;
(E)-4-((1,3-bis(2-fluorobenzyl)-1,2,3,6-tetrahydro-2,6-dioxo-9H-purin-8-yl)cinnamic Acid Nonaethylene Glycol Methyl Ether Ester;
(E)-4-((1,3-bisphenethyl)-1,2,3,6-tetrahydro-2,6-dioxo-9H-purin-8-yl)cinnamic Acid Nonaethylene Glycol Methyl Ether Ester;
(E)-4-((1-cyclohexyl methyl-3-methyl)-1,2,3,6-tetrahydro-2,6-dioxo-9H-purin-8-yl)cinnamic Acid Nonaethylene Glycol Methyl Ether Ester;
(E)-4-((1-H-3-(2-methyl-propyl)-1,2,3,6-tetrahydro-2,6-dioxo-9H-purin-8-yl)cinnamic Acid Nonaethylene Glycol Methyl Ether Ester;
(E)-4-(1,3-bis(4-fluorobenzyl)-1,2,3,6-tetrahydro-2,6-dioxo-9H-purin-8-yl)cinnamic Acid Nonaethylene Glycol Methyl Ether Ester;
(E)-4-(1,3-bis(cyctohexylmethyl)-1,2,3,6-tetrahydro-2,6-dioxo-9H-purin-8-yl)cinnamic Acid Hexaethylene Glycol dodecyl Ether Ester;
(E)-4-(1,3-bis(cyclobutylmethyl)-1,2,3,6-tetrahydro-2,6-dioxo-9H-purin-8-yl)cinnamic Acid Nonaethylene Glycol Methyl Ether Ester;
(E)-4-(1-methyl-3-cyclohexylmethyl)-1,2,3,6-tetrahydro-2,6-dioxo-9H-purin-8-yl)cinnamic Acid Nonaethylene Glycol Methyl Ether Ester;
(E)-4-(1-methyl-3-isobutyl)-1,2,3,6-tetrahydro-2,6-dioxo-9H-purin-8-yl)cinnamic Acid Nonaethylene Glycol Methyl Ether Ester;
4-(1,3-bis(cyclohexylmethyl)-1,2,3,6-tetrahydro-2,6-dioxo-9H-purin-8-yl)benzoic Acid Nonaethylene Glycol Methyl Ether Ester;
(E)-4-(1,3-bis(cyclohexyl)-1,2,3,6-tetrahydro-2,6-dioxo-9H-purin-8-yl)cinnamic Acid Nonaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Nonaethylene Glycol Methyl Ether Amide;
(E)-4-(1,3-bis(cyclopentylmethyl)-1,2,3,6-tetrahydro-6-oxo-2-thioxo-9H-purin-8-yl)cinnamic Acid Nonaethylene Glycol Methyl Ether Amide;
(E)-4-(1,3-bis(2-methyl-propyl)-1,2,3,6-tetrahydro-2,6-dioxo-9H-purin-8-yl)cinnamic Acid Nonaethylene Glycol Methyl Ether Amide;
(E)-4-((1-cyclohexylmethyl-3-propyl)-1,2,3,6-tetrahydro-2,6-dioxo-9H-purin-8-yl)cinnamic Acid Nonaethylene Glycol Methyl Ether Amide;
4-(1,3-Bis(cyclohexylmethyl)-1,2,3,6-tetrahydro-2,6-dioxo-9H-purin-8-yl)benzoic Acid Nonaethylene Glycol Methyl Ether Amide;
4-(1,3-Bis(cyclohexylmethyl)-1,2,3,6-tetrahydro-2,6-dioxo-9H-purin-8-yl)benzoic Acid-N-methyl-Nonaethylene Glycol Methyl Ether Amide;
(E)-4-(1,3-Bis(cyclohexylmethyl)-2,3,6,7-tetrahydro-2,6-dioxo-7-benzyl-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-oxo-2-phenylethyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-methyl-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-propynyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo7-(2-oxo-2-methylethyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(3-morpholinopropyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-ethyl-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-ethoxy-2-oxoethyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(2-methyl-2-propenyl)-1H-purin-8-yl)cinnamic Acid Nonaethylene Glycol Methyl Ether Ester;
(E)-4-(1,3-Bis(cyclohexylmethyl)-2,3,6,7-tetrahydro-2,6-dioxo-7-(cyanomethyl)-1H-purin-8-yl)cinnamic Acid Nonaethylene Glycol Methyl Ether Ester;
4-(1,3-Bis(cyclohexylmethyl)-2,3,6,7-tetrahydro-2,6-dioxo-7-benzyl-1H-purin-8-yl)benzoic Acid Nonaethylene Glycol Methyl Ether Ester;
4-(1,3-Bis(cyclohexylmethyl)-2,3,6,7-tetrahydro-2,6-dioxo-7-methyl-1H-purin-8-yl)benzoic Acid Nonaethylene Glycol Methyl Ether Ester;
4-[(1,3-Bis(cyclohexylmethyl)-2,3,6,7-tetrahydro-2,6-dioxo-7-methyl-1H-purin-8-yl)phenyl] propionic Acid Nonaethylene Glycol Methyl Ether Ester;
(E)-4-(1,3-Bis(cydohexylmethyl)-2,3,6;.7-tetrahydro-2,6-dioxo-7-methyl-1H-purin-8-yl)cinnamic Acid Nonaethylene Glycol Methyl Ether Amide;
(E)-4-(1,3-Bis(cyclohexylmethyl)-2,3,6,7-tetrahydro-2,6-dioxo-7-benzyl-1H-purin-8-yl)cinnamic Acid Nonaethylene Glycol Methyl Ether Amide;
4-(1,3-Bis(cyclohexylmethyl)-2,3,6,7-tetrahydro-2,6-dioxo-7-benzyl-1H-purin-8-yl)benzoic Acid Nonaethylene Glycol Methyl Ether Amide;
4-(1,3-Bis(cyclohexylmethyl)-2,3,6,7-tetrahydro-2,6-dioxo-7-methyl-1H-purin-8-yl)benzoic Acid Nonaethylene Glycol Methyl Ether Amide;
1,3-Bis(cyclohexylmethyl)-8-[4-(2,5,8,11,14,17,20,23,26,29-decaoxatriacont-1-yl)phenyl]-3,7-dihydro-1H-purine-2,6-dione;
(E)-3-[5-[1,3-bis(cydohexylmethyl)-2,3,6,7-tetrahydro-2,6-dioxo-1H-purin-8-yl]-2-thienyl]-2-propenoic Acid Nonaethylene Glycol Methyl Ether Ester;
6-(1,3-Bis(cyclohexylmethyl)-1,2,3,6-tetrahydro-2,6-dioxo-9H-purin-8-yl)nicotinic Acid Nonaethylene Glycol Methyl Ether Amide;
(E)-3-(1,3-Bis(cyclohexylmethyl)-1,2,3,6-tetrahydro-2,6-dioxo-9H-purin-8-yl)cinnamic Acid N-cyclopropylmethyl Nonaethylene Glycol Methyl Ether Amide ;
(E)-4-(1,3-Bis(cyclohexylmethyl)-1,2,3,6-tetrahydro-2,6-dioxo-9H-purin-8-yl)cinnamic Acid Hexaethylene Glycol Benzyl Ether Amide;
(E)-4-[(3-Cyclohexylmethyl)-2,3,6,7-tetrahydro-2,6-dioxo-7-methyl-1H-purin-8-yl]cinnamic Acid Heptaethylene Glycol Methyl Ether Ester;
(E)-4-[(3-Cyclohexylmethyl)-2,3,6,7-tetrahydro-2,6-dioxo-7-methyl-1H-purin-8-yl]-cinnamic Acid Nonaethylene Glycol Methyl Ether Ester;
(E)-4-[(3-Cyclohexylmethyl)-2,3,6,7-tetrahydro-2,6-dioxo-1,7-dimethyl-1H-purin-8-yl]cinnamic Acid Nonaethylene Glycol Methyl Ether Ester;
4-[1,3-Bis(cyclohexylmethyl)-1,2,3,6-tetrahydro-2,6-dioxo-9H-purin-8-yl]benzylamine Heptaethylene Glycol Methyl Ether;
4-[1,3-Bis(cyclohexylmethyl)-1,2,3,6-tetrahydro-2,6-dioxo-9H-purin-8-yl]benzylamine N-Heptaethylene Glycol Methyl Ether Hydrochloride;
4-[1,3-Bis(cyclohexylmethyl)-1,2,3,6-tetrahydro-2,6-dioxo-9H-purin-8-yl]benzylamine N-Nonaethylene Glycol Methyl Ether;
1,3-Bis(cyclohexylmethyl)-8-[3-(2,5,8,11,14,17,20,23,26,29-decaoxatriacont-1-yl)phenyl]-3,7-dihydro-1H-purine-2,6-dione;
(E)-4-(1,3-Bis(cyclohexylmethyl)-2,3,6,7-tetrahydro-2,6-dioxo-7-methyl-1H-purin-8-yl)cinnamic Acid Heptaethylene Glycol Methyl Ether Ester;
(E)-4-(1,3-Bis(cyclohexylmethyl)-2,3,6,7-tetrahydro-2,6-dioxo-7-methyl-1H-purin-8-yl)cinnamic Acid Pentaethylene Glycol Methyl Ether Ester;
(E)-4-(1,3-Bis(cyclohexylmethyl)-2,3,6,7-tetrahydro-2,6-dioxo-7-propyl-1H-purin-8-yl)cinnamic Acid Nonaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Decaethylene Glycol Methyl Ether Ester;
(E)-4-(1,3-Bis(cyclohexylmethyl)-1,2,3,6-tetrahydro-2,6-dioxo-9H-purin-8-yl)cinnamic Acid Nonaethylene Glycol Methyl Ether Ester;
(E)-3-[1,3-Bis(cyclohexylmethyl)-1,2,3,6-tetrahydro-2,6-dioxo-9H-purin-8-yl]cinnamic Acid Nonaethylene Glycol Methyl Ether Ester;
(E)-4-[1,3-Bis(cyclohexylmethyl)-1,2,3,6-tetrahydro-2,6-dioxo-9H-purin-8-yl)cinnamic acid Nonaethylene Glycol Methyl Ether Amide; and
(E)-4-[1,3-Bis(cyclohexylmethyl)-1,2,3,6-tetrahydro-2,6-dioxo-9H-purin-8-yl]benzoic acid Nonaethylene Glycol Methyl Ether Ester; and
pharmaceutically acceptable solvates thereof.

4. The use according to claim 1 or 2 wherein the compound of formula (I) is (E)-4-(1,3-bis(cyclohexylmethyl)-1,2,3,6-tetrahydro-2,6-dioxo-9H-purin-8-yl)cinnamic acid nonaethylene glycol methyl ether ester or a pharmaceutically acceptable solvate thereof.

## Patentansprüche

1. Verwendung einer Verbindung der Formel (I): 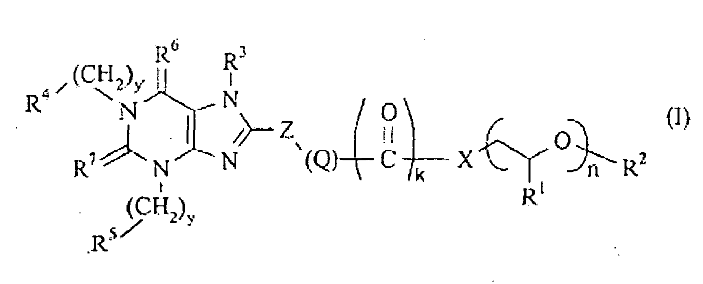 wobei:
Z ausgewählt ist aus C₅₋₆-Cycloalkyl, C₆-Aryl, substituiertem C₅₋₆-Cycloalkyl, substituiertem C₆-Aryl, 5- oder 6-gliedrigen heterocyclischen Resten, substituierten 5- oder 6-gliedrigen heterocyclischen Resten, 5- oder 6-gliedrigem Heteroaryl und substituiertem 5- oder 6-gliedrigem Heteroaryl;
R¹ H oder Methyl ist;
R² H, C₁₋₁₂-Alkyl, Aryl oder Aralkyl ist;
k 0 oder 1 ist;
n eine ganze Zahl von 1 bis 50 ist;
X ausgewählt ist aus
-O-,
-N(H)-,
-N(C₁₋₆-Alkyl)-,
-N(C₃₋₈-Cycloalkyl)-,
-N(C₁₋₈-Alkyl)(C₃₋₈-cycloalkyl), und
-N[(CH₂CH₂O)ₘ(C₁₋₁₂-Alkyl, Aryl oder Aralkyl)]-;
m 0 bis 12 ist;
Q ausgewählt ist aus (-CH₂)ₚ, (-CH=CH-)ₚ, (-C≡C-)ₚ, (-OCH₂-)ₚ und (-CH₂O)ₚ, wobei p 0 bis 4 ist;
y und y' jeweils unabhängig 0 bis 10 sind;
R³ ausgewählt ist aus
H; geradkettigem oder verzweigtem C₁₋₁₂-Alkyl, wobei das Alkyl gegebenenfalls mit einem funktionellen Rest, ausgewählt aus Phenyl, -CO-Phenyl, CN, -CO(C₁₋₃)Alkyl, -CO₂(C₁₋₃-Alkyl) substituiert sein kann und wobei das C₁₋₁₂-Alkyl gegebenenfalls ein oder mehr O-Atome in der Alkylkette aufweisen kann;
geradkettigem oder verzweigtem C₂₋₆-Alkenyl;
geradkettigem oder verzweigtem C₂₋₆-Alkinyl; und
-C₁₋₃-Alkyl-NR⁸R⁹, wobei R⁸ und R⁹ jeweils unabhängig ausgewählt sind aus H und C₁₋₃-Alkyl oder R⁸ und R⁹ zusammen mit dem N, an das sie gebunden sind, einen 5- oder 6-gliedrigen heterocyclischen Rest bilden, welcher gegebenenfalls 1 bis 2 andere Heteroatome enthält, ausgewählt aus O, N und S;
R⁴ und R⁵ jeweils unabhängig ausgewählt sind aus
C₃₋₈-Cycloalkyl,
geradkettigem oder verzweigtem C₁₋₆-Alkyl,
H,
geradkettigem oder verzweigtem C₂₋₆-Alkenyl,
Aryl,
substituiertem Aryl,
heterocyclischen Resten,
substituierten heterocyclischen Resten,
Heteroaryl und
substituiertem Heteroaryl; und
R⁶ und R⁷ jeweils unabhängig O oder S sind; wobei der Begriff "Aralkyl" ein C₁₋₁₂-Alkyl betrifft, welches ein geradkettiger oder verzweigter Alkylrest sein kann, der mit einem Aryl- oder einem substituierten Arylrest substituiert sein kann, und der Begriff "Alkyl" geradkettige oder verzweigte Kohlenwasserstoffketten darstellt, welche die angegebene Anzahl an Kohlenstoffatomen enthalten,
oder eines pharmazeutisch verträglichen Solvats davon, zur Herstellung eines Medikaments zur Behandlung oder Prophylaxe von Reizkolon in einem Lebewesen.

2. Verwendung gemäß Anspruch 1, wobei die Verbindung der Formel (I) eine Verbindung der Formel (I-A) ist 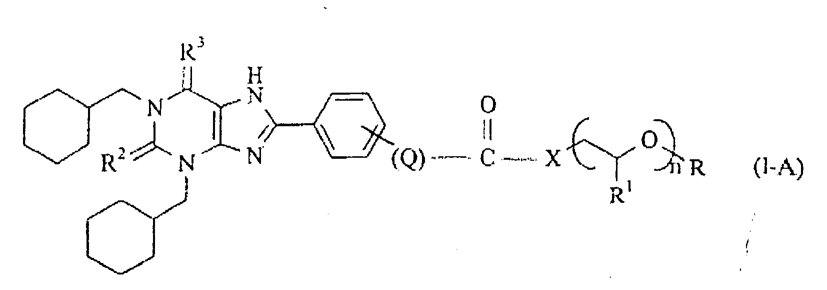 wobei:
X -O- oder -NH- ist;
Q ausgewählt ist aus (-CH₂-)ₚ, (-CH=CH-)ₚ und (-C≡C-)ₚ, wobei p 0 bis 4 ist;
R¹ H oder Methyl ist;
R² und R³ jeweils unabhängig O oder S sind;
n eine ganze Zahl von 1 bis 50 ist; und
R H oder Methyl ist;
oder eines pharmazeutisch verträglichen Solvats davon.

3. Verwendung gemäß Anspruch 1, wobei die Verbindung der Formel (I) ausgewählt ist aus:
(E)-4-(1,3-Bis(benzyl)-1,2,3,6-tetrahydro-2,6-dioxo-9H-purin-8-yl)zimtsäure-nonaethylenglycolmethyletherester;
(E)-4-[1,3-Bis(cyclohexylmethyl)-1,2,3,6-tetrahydro-2,6-dioxo-9H-purin-8-yl]zimtsäure-nonaethylenglycolmethyletherester;
(E)-4-(1,3-Bis(cyclopentylmethyl)-1,2,3,6-tetrahydro-2,6-dioxo-9H-purin-8-yl)zimtsäure-nonaethylenglycolmethyletherester;
(E)-4-(1,3-Bis(propyl)-1,2,3,6-tetrahydro-2,6-dioxo-9H-purin-8-yl)zimtsäure-nonaethylenglycolmethyletherester;
(E)-4-(1,3-Bis(cyclopropylmethyl)-1,2,3,6-tetrahydro-2,6-dioxo-9H-purin-8-yl)zimtsäure-nonaethylenglycolmethyletherester;
(E)-3-((1-Propyl-3-benzyl)-1,2,3,6-tetrahydro-2,6-dioxo-9H-purin-8-yl)zimtsäure-nonaethylenglycolmethyletherester;
(E)-4-(1,3-Bis(cycloheptylmethyl)-1,2,3,6-tetrahydro-2,6-dioxo-9H-purin-8-yl)-zimtsäure-nonaethylenglycolmethyletherester;
(E)-4-(1,3-Bis(cyclohexylethyl)-1,2,3,6-tetrahydro-2,6-dioxo-9H-purin-8-yl)zimtsäure-nonaethylenglycolmethyletherester;
(E)-4-(1,3-Bis(phenyl)-1,2,3,6-tetrahydro-2,6-dioxo-9H-purin-8-yl)zimtsäure-nonaethylenglycolmethyletherester;
(E)-4-(1,3-Bis(2-methylpropyl)-1,2,3,6-tetrahydro-2,6-dioxo-9H-purin-8-yl)zimtsäure-nonaethylenglycolmethyletherester;
(E)-4-((1-Propyl-3-cyclohexylmethyl)-1,2,3,6-tetrahydro-2,6-dioxo-9H-purin-8-yl)zimtsäure-nonaethylenglycolmethyletherester;
(E)-4-(1,3-Bis(bicyclo(2.2.1)hept-2-ylmethyl)-1;2,3,6-tetrahydro-2,6-dioxo-9H-purin-8-yl)zimtsäure-nonaethylenglycolmethyletherester;
(E)-4-(1-Cyclohexylmethyl-3-butyl)-1,2,3,6-tetrahydro-2,6-dioxo-9H-purin-8-yl)zimtsäure-nonaethylenglycolmethyletherester;
(E)-4-((1-Cyclohexylmethyl-3-propyl)-1,2,3,6-tetrahydro-2,6-dioxo-9H-purin-8-yl)zimtsäure-nonaethylenglycolmethyletherester;
(E)-4-(1,3-Bis(benzyl)-1,2,3,6-tetrahydro-2-thioxo-6-oxo-9H-purin-8-yl)zimtsäure-nonaethylenglycolmethyletherester;
(E)-4-(1-Methyl-3-(3-cyanobenzyl))-1,2,3,6-tetrahydro-2,6-dioxo-9H-purin-8-yl)zimtsäure-nonaethylenglycolmethyletherester;
(E)-4-((1,3-Bis(3-fluorbenzyl)-1,2,3,6-tetrahydro-2,6-dioxo-9H-purin-8-yl)zimtsäure-nonaethylenglycolmethyletherester;
(E)-4-((1,3-Bis(2-fluorbenzyl)-1,2,3,6-tetrahydro-2,6-dioxo-9H-purin-8-yl)zimtsäure-nonaethylenglycolmethyletherester;
(E)-4-((1,3-Bisphenethyl)-1,2,3,6-tetrahydro-2,6-dioxo-9H-purin-8-yl)zimtsäure-nonaethylenglycolmethyletherester;
(E)-4-((1-Cyclohexylmethyl-3-methyl)-1,2,3,6-tetrahydro-2,6-dioxo-9H-purin-8-yl)zimtsäure-nonaethylenglycolmethyletherester;
(E)-4-((1-H-3-(2-methylpropyl)-1,2,3,6-tetrahydro-2,6-dioxo-9H-purin-8-yl)zimtsäure-nonaethylenglycolmethyletherester;
(E)-4-(1,3-Bis(4-fluorbenzyl)-1,2,3,6-tetrahydro-2,6-dioxo-9H-purin-8-yl)zimtsäure-nonaethylenglycolmethyletherester;
(E)-4-(1,3-Bis(cyclohexylmethyl)-1,2,3,6-tetrahydro-2,6-dioxo-9H-purin-8-yl)zimtsäure-hexaethylenglycoldodecyletherester;
(E)-4-(1,3-Bis(cyclobutylmethyl)-1,2,3,6-tetrahydro-2,6-dioxo-9H-purin-8-yl)zimtsäure-nonaethylenglycolmethyletherester;
(E)-4-(1-Methyl-3-cyclohexylmethyl)-1,2,3,6-tetrahydro-2,6-dioxo-9H-purin-8-yl)zimtsäure-nonaethylenglycolmethyletherester;
(E)-4-(1-Methyl-3-isobutyl)-1,2,3,6-tetrahydro-2,6-dioxo-9H-purin-8-yl)zimtsäure-nonaethylenglycolmethyletherester;
4-(1,3-Bis(cyclohexylmethyl)-1,2,3,6-tetrahydro-2,6-dioxo-9H-purin-8-yl)benzoesäure-nonaethylenglycolmethyletherester;
(E)-4-(1,3-Bis(cyclohexyl)-1,2,3,6-tetrahydro-2,6-dioxo-9H-purin-8-yl)zimtsäure-nonaethylenglycolmethyletherester;
(E)-4-(1,3-Bis(cyclohexylmethyl)-1,2,3,6-tetrahydro-2,6-dioxo-9H-purin-8-yl)zimtsäure-nonaethylenglycolmethyletheramid;
(E)-4-(1,3-Bis(cyclopentylmethyl)-1,2,3,6-tetrahydro-6-oxo-2-thioxo-9H-purin-8-yl)zimtsäure-nonaethylenglycolmethyletheramid;
(E)-4-(1,3-Bis(2-methylpropyl)-1,2,3,6-tetrahydro-2,6-dioxo-9H-purin-8-yl)zimtsäure-nonaethylenglycolmethyletheramid;
(E)-4-((1-Cyclohexylmethyl-3-propyl)-1,2,3,6-tetrahydro-2,6-dioxo-9H-purin-8-yl)zimtsäure-nonaethylenglycolmethyletheramid;
4-(1,3-Bis(cyclohexylmethyl)-1,2,3,6-tetrahydro-2,6-dioxo-9H-purin-8-yl)benzoesäure-nonaethylenglycolmethyletheramid;
4-(1,3-Bis(cyclohexylmethyl)-1,2,3,6-tetrahydro-2,6-dioxo-9H-purin-8-yl)benzoesäure-N-methylnonaethylenglycolmethyletheramid;
(E)-4-(1,3-Bis(cyclohexylmethyl)-2,3,6,7-tetrahydro-2,6-dioxo-7-benzyl-1H-purin-8-yl)zimtsäure-nonaethylenglycolmethyletherester;
(E)-4-(1,3-Bis(cyclohexylmethyl)-2,3,6,7-tetrahydro-2,6-dioxo-7-(2-oxo-2-phenylethyl)-1H-purin-8-yl)zimtsäure-nonaethylenglycolmethyletherester;
(E)-4-(1,3-Bis(cyclohexylmethyl)-2,3,6,7-tetrahydro-2,6-dioxo-7-methyl-1H-purin-8-yl)zimtsäure-nonaethylenglycolmethyletherester;
(E)-4-(1,3-Bis(cyclohexylmethyl)-2,3,6,7-tetrahydro-2,6-dioxo-7-(2-propinyl)-1H-purin-8-yl)zimtsäure-nonaethylenglycolmethyletherester;
(E)-4-(1,3-Bis(cyclohexylmethyl)-2,3,6,7-tetrahydro-2,6-dioxo-7-(2-oxo-2-methylethyl)-1H-purin-8-yl)zimtsäure-nonaethylenglycolmethyletherester;
(E)-4-(1,3-Bis(cyclohexylmethyl)-2,3,6,7-tetrahydro-2,6-dioxo-7-(3-morpholinopropyl)-1H-purin-8-yl)zimtsäure-nonaethylenglycolmethyletherester;
(E)-4-(1,3-Bis(cyclohexylmethyl)-2,3,6,7-tetrahydro-2,6-dioxo-7-ethyl-1H-purin-8-yl)zimtsäure-nonaethylenglycolmethyletherester;
(E)-4-(1,3-Bis(cyclohexylmethyl)-2,3,6,7-tetrahydro-2,6-dioxo-7-(2-ethoxy-2-oxoethyl)-1H-purin-8-yl)zimtsäure-nonaethylenglycolmethyletherester;
(E)-4-(1,3-Bis(cyclohexylmethyl)-2,3,6,7-tetrahydro-2,6-dioxo-7-(2-methyl-2-propenyl)-1H-purin-8-yl)zimtsäure-nonaethylenglycolmethyletherester;
(E)-4-(1,3-Bis(cyclohexylmethyl)-2,3,6,7-tetrahydro-2,6-dioxo-7-(cyanomethyl)-1H-purin-8-yl)zimtsäure-nonaethylenglycolmethyletherester;
4-(1,3-Bis(cyclohexylmethyl)-2,3,6,7-tetrahydro-2,6-dioxo-7-benzyl-1H-purin-8-yl)benzoesäure-nonaethylenglycolmethyletherester;
4-(1,3-Bis(cyclohexylmethyl)-2,3,6,7-tetrahydro-2,6-dioxo-7-methyl-1H-purin-8-yl)benzoesäure-nonaethylenglycolmethyletherester;
4-[(1,3-Bis(cyclohexylmethyl)-2,3,6,7-tetrahydro-2,6-dioxo-7-methyl-1H-purin-8-yl)-phenyl]propionsäure-nonaethylenglycolmethyletherester;
(E)-4-(1,3-Bis(cyclohexylmethyl)-2,3,6,7-tetrahydro-2,6-dioxo-7-methyl-1H-purin-8-yl)zimtsäure-nonaethylenglycolmethyletheramid;
(E)-4-(1,3-Bis(cyclohexylmethyl)-2,3,6,7-tetrahydro-2,6-dioxo-7-benzyl-1H-purin-8-yl)zimtsäure-nonaethylenglycolmethyletheramid;
4-(1,3-Bis(cyclohexylmethyl)-2,3,6,7-tetrahydro-2,6-dioxo-7-benzyl-1H-purin-8-yl)-benzoesäure-nonaethylenglycolmethyletheramid;
4-(1,3-Bis(cyclohexylmethyl)-2,3,6,7-tetrahydro-2,6-dioxo-7-methyl-1H-purin-8-yl)-benzoesäure-nonaethylenglycolmethyletheramid;
1,3-Bis(cyclohexylmethyl)-8-[4-(2,5,8,11,14,17,20,23,26,29-decaoxatriacont-1-yl)phenyl]-3,7-dihydro-1H-purin-2,6-dion;
(E)-3-[5-[1,3-Bis(cyclohexylmethyl)-2,3,6,7-tetrahydro-2,6-dioxo-1H-purin-8-yl]2-thienyl]-2-propensäure-nonaethylenglycolmethyletherester;
6-(1,3-Bis(cyclohexylmethyl)-1,2,3,6-tetrahydro-2,6-dioxo-9H-purin-8-yl)nikotinsäure-nonaethylenglycolmethyletheramid;
(E)-3-(1,3-Bis(cyclohexylmethyl)-1,2,3,6-tetrahydro-2,6-dioxo-9H-purin-8-yl)zimtsäure-N-cyclopropylmethyl-nonaethylenglycolmethyletheramid;
(E)-4-(1,3-Bis(cyclohexylmethyl)-1,2,3,6-tetrahydro-2,6-dioxo-9H-purin-8-yl)zimtsäure-hexaethylenglycolbenzyletheramid;
(E)-4-[(3-Cyclohexylmethyl)-2,3,6,7-tetrahydro-2,6-dioxo-7-methyl- 1H-purin-8-yl] zimtsäure-heptaethylenglycolmethyletherester;
(E)-4-[(3-Cyclohexylmethyl)-2,3,6,7-tetrahydro-2,6-dioxo-7-methyl-1H-purin-8-yl]zimtsäure-nonaethylenglycolmethyletherester;
(E)-4-[(3-Cyclohexylmethyl)-2,3,6,7-tetrahydro-2,6-dioxo-1,7-dimethyl-1H-purin-8-yl]-zimtsäure-nonaethylenglycolmethyletherester;
4-[1,3-Bis(cyclohexylmethyl)-1,2,3,6-tetrahydro-2,6-dioxo-9H-purin-8-yl]benzylaminheptaethylenglycolmethylether;
4-[1,3-Bis(cyclohexylmethyl)-1,2,3,6-tetrahydro-2,6-dioxo-9H-purin-8-yl]benzylamin-N-heptaethylenglycolmethyletherhydrochlorid;
4-[1,3-Bis(cyclohexylmethyl)-1,2,3,6-tetrahydro-2,6-dioxo-9H-purin-8-yl]benzylamin-N-nonaethylenglycolmethylether;
1,3-Bis(cyclohexylmethyl)-8-[3-(2,5,8,11,14,17,20,23,26,29-decaoxatriacont-1-yl)phenyl]-3,7-dihydro-1H-purin-2,6-dion;
(E)-4-(1,3-Bis(cyclohexylmethyl)-2,3,6,7-tetrahydro-2,6-dioxo-7-methyl-1H-purin-8-yl)zimtsäure-heptaethylenglycolmethyletherester;
(E)-4-(1,3-Bis(cyclohexylmethyl)-2,3,6,7-tetrahydro-2,6-dioxo-7-methyl-1H-purin-8-yl)zimtsäure-pentaethylenglycolmethyletherester;
(E)-4-(1,3-Bis(cyclohexylmethyl)-2,3,6,7-tetrahydro-2,6-dioxo-7-propyl-1H-purin-8-yl)zimtsäure-nonaethylenglycolmethyletherester;
(E)-4-(1,3-Bis(cyclohexylmethyl)-1,2,3,6-tetrahydro-2,6-dioxo-9H-purin-8-yl)zimtsäure-decaethylenglycolmethyletherester;
(E)-4-(1,3-Bis(cyclohexylmethyl)-1,2,3,6-tetrahydro-2,6-dioxo-9H-purin-8-yl)zimtsäure-nonaethylenglycolmethyletherester;
(E)-3-[1,3-Bis(cyclohexylmethyl)-1,2,3,6-tetrahydro-2,6-dioxo-9H-purin-8-yl]zimtsäure-nonaethylenglycolmethyletherester;
(E)-4-[1,3-Bis(cyclohexylmethyl)-1,2,3,6-tetrahydro-2,6-dioxo-9H-purin-8-yl)zimtsäure-nonaethylenglycolmethyletheramid; und
(E)-4-[1,3-Bis(cyclohexylmethyl)-1,2,3,6-tetrahydro-2,6-dioxo-9H-purin-8-yl)benzoesäure-nonaethylenglycolmethyletherester; und
pharmazeutisch verträglichen Solvaten davon.

4. Verwendung gemäß Anspruch 1 oder 2, wobei die Verbindung der Formel (I) (E)-4-(1,3-Bis(cyclohexylmethyl)-1,2,3,6-tetrahydro-2,6-dioxo-9H-purin-8-yl)zimtsäure-nonaethylenglycolmethyletherester oder ein pharmazeutisch verträgliches Solvat davon ist.

## Revendications

1. Utilisation d'un composé de formule (I) : 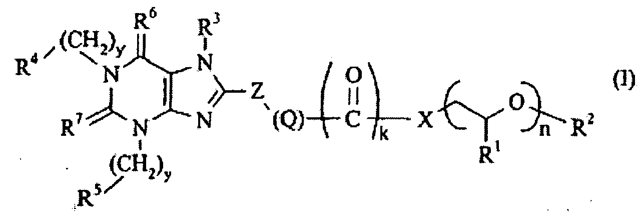 dans laquelle :
Z est choisi dans le groupe consistant en des groupes cycloalkyle en C₅ ou C₆, aryle en C₆, cycloalkyle en C₅ ou C₆ substitué, aryle en C₆ substitué, hétérocycliques penta- ou hexagonaux, hétérocycliques penta- ou hexagonaux substitués, hétéroaryle penta- ou hexagonal et hétéroaryle penta- ou hexagonal substitué
R¹ représente H ou un groupe méthyle ;
R² représente H ou un groupe alkyle en C₁ à C₁₂, aryle ou aralkyle ;
k est égal à 0 ou 1 ;
n représente un nombre entier de 1 à 50 ;
X est choisi dans le groupe consistant en des groupes
-O-,
-N(H)- ,
-N(alkyle en C₁ à C₆)-,
-N(cycloalkyle en C₃ à C₈)-,
-N(alkyle en C₁ à C₈) (cycloalkyle en C₃ à C₈), et
-N[(CH₂CH₂O)ₘ (alkyle en C₁ à C₁₂, aryle ou aralkyle)] -;
m a une valeur de 0 à 12 ;
Q est choisi dans le groupe consistant en des groupes (-CH₂)ₚ, (-CH=CH-)ₚ, (-C≡C-)ₚ, (-OCH₂-)ₚ et (-CH₂O)ₚ dans lesquels p a une valeur de 0 à 4 ;
y et y' ont chacun indépendamment une valeur de 0 à 10 ;
R³ est choisi dans le groupe consistant en
H ;
un groupe alkyle en C₁ à C₁₂ droit ou ramifié, ledit groupe alkyle pouvant être facultativement substitué avec un groupe fonctionnel choisi dans le groupe consistant en des groupes phényle, -CO-phényle, CN, -CO (alkyle en C₁ à C₃), -CO₂(alkyle en C₁ à C₃), et ledit groupe alkyle en C₁ à C₁₂ pouvant comprendre facultativement un ou plusieurs atomes de O dans la chaîne alkyle ;
un groupe alcényle en C₂ à C₆ droit ou ramifié ;
un groupe alcynyle en C₂ à C₆ droit ou ramifié ; et
un groupe (alkyle en C₁ à C₃) NR⁸R⁹ dans lequel R⁸ et R⁹ sont choisis chacun indépendamment dans le groupe consistant en H et un groupe alkyle en C₁ à C₃, ou bien R⁸ et R⁹, conjointement avec l'atome de N auquel ils sont liés, forment un groupe hétérocyclique penta- ou hexagonal contenant facultativement 1 ou 2 autres hétéroatomes choisis dans le groupe consistant en O, N et S ;
R⁴ et R⁵ sont choisis chacun indépendamment dans le groupe consistant en des groupes
- cycloalkyle en C₃ à C₈,
- alkyle en C₁ à C₆ droit ou ramifié,
- H,
- alcényle en C₂ à C₆ droit ou ramifié,
- aryle,
- aryle substitué,
- hétérocycliques,
- hétérocycliques substitués,
- hétéroaryle et
- hétéroaryle substitué ; et
R⁶ et R⁷ représentent chacun indépendamment O ou S ; lorsque le terme "aralkyle" désigne un groupe alkyle en C₁ à C₁₂ qui peut être un groupe alkyle droit ou ramifié qui est substitué avec un groupe aryle ou groupe aryle substitué et le terme "alkyle" désigne des chaînes hydrocarbonées droites ou ramifiées contenant le nombre indiqué d'atomes de carbone,
ou d'un de ses produits de solvatation pharmaceutiquement acceptables, pour la préparation d'un médicament destiné au traitement ou à la prophylaxie du syndrome du côlon irritable chez un animal.

2. Utilisation suivant la revendication 1, dans laquelle le composé de formule (I) est un composé de formule (I-A) 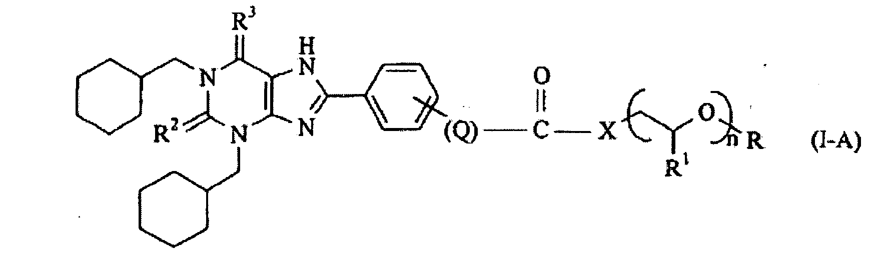 dans laquelle:
X représente un groupe -O- ou -NH- ;
Q est choisi dans le groupe consistant en des groupes (-CH₂-)ₚ, (-CH=CH-)ₚ et (-C≡C-)ₚ dans lesquels p a une valeur de 0 à 4 ;
R¹ représente H ou un groupe méthyle ;
R² et R³ représentent chacun indépendamment O ou S ;
n représente un nombre entier de 1 à 50 ; et
R représente H ou un groupe méthyle ;
ou un de ses produits de solvatation pharmaceutiquement acceptables.

3. Utilisation suivant la revendication 1, dans laquelle le composé de formule (I) est choisi dans le groupe consistant en :
ester d'éther méthylique de nonaéthylèneglycol d'acide (E)-4-(1,3-bis(benzyl)-1,2,3,6-tétrahydro-2,6-dioxo-9H-purine-8-yl)cinnamique ;
ester d'éther méthylique de nonaéthylèneglycol d'acide (E)-4-[1,3-bis(cyclohexylméthyl)-1,2,3,6-tétrahydro-2,6-dioxo-9H-purine-8-yl]cinnamique ;
ester d'éther méthylique de nonaéthylèneglycol d'acide (E)-4-[1,3-bis(cyclopentylméthyl)-1,2,3,6-tétrahydro-2,6-dioxo-9H-purine-8-yl]cinnamique ;
ester d'éther méthylique de nonaéthylèneglycol d'acide (E)-4-(1,3-bis(propyl)-1,2,3,6-tétrahydro-2,6-dioxo-9H-purine-8-yl)cinnamique ;
ester d'éther méthylique de nonaéthylèneglycol d'acide (E)-4-(1,3-bis(cyclopropylméthyl)-1,2,3,6-tétrahydro-2,6-dioxo-9H-purine-8-yl)cinnamique ;
ester d'éther méthylique de nonaéthylèneglycol d'acide (E)-3-((1-propyl-3-benzyl)-1,2,3,6-tétrahydro-2,6-.dioxo-9H-purine-8-yl)cinnamigue ;
ester d'éther méthylique de nonaéthylèneglycol d'acide (E)-4-(1,3-bis(cycloheptylméthyl)-1,2,3,6-tétrahydro-2,6-dioxo-9H-purine-8-yl)cinnamique ;
ester d'éther méthylique de nonaéthylèneglycol d'acide (E)-4-(1,3-bis(cyclohexyléthyl)-1,2,3,6-tétrahydro-2,6-dioxo-9H-purine-8-yl)cinnamique ;
ester d'éther méthylique de nonaéthylèneglycol d'acide (E)-4-(1,3-bis(phényl)-1,2,3,6-tétrahydro-2,6-dioxo-9H-purine-8-yl)cinnamique ;
ester d'éther méthylique de nonaéthylèneglycol d'acide (E)-4-(1,3-bis(2-méthyl-propyl)-1,2,3,6-tétrahydro-2,6-dioxo-9H-purine-8-yl)cinnamique ;
ester d'éther méthylique de nonaéthylèneglycol d'acide (E)-4-((1-propyl-3-cyclohexylméthyl)-1,2,3,6-tétrahydro-2,6-dioxo-9H-purine-8-yl)cinnamique ;
ester d'éther méthylique de nonaéthylèneglycol d'acide (E)-4-(1,3-bis(bicyclo(2.2.1)hept-2-ylméthyl)-1,2,3,6-tétrahydro-2,6-dioxo-9H-purine-8-yl)cinnamique ;
ester d'éther méthylique de nonaéthylèneglycol d'acide (E)-4-(1-cyclohexylméthyl-3-butyl)-1,2,3,6-tétrahydro-2,6-dioxo-9H-purine-8-yl)cinnamique ;
ester d'éther méthylique de nonaéthylèneglycol d'acide (E)-4-((1-cyclohexylméthyl-3-propyl)-1,2,3,6-tétrahydro-2,6-dioxo-9H-purine-8-yl)cinnamique ;
ester d'éther méthylique de nonaéthylèneglycol d'acide (E)-4-(1,3-bis(benzyl)-1,2,3,6-tétrahydro-2-thioxo-6-oxo-9H-purine-8-yl)cinnamique ;
ester d'éther méthylique de nonaéthylèneglycol d'acide (E)-4-(1-méthyl-3-(3-cyanobenzyl))-1,2,3,6-tétrahydro-2,6-dioxo-9H-purine-8-yl)cinnamique ;
ester d'éther méthylique de nonaéthylèneglycol d'acide (E)-4-((1,3-bis(3-fluorobenzyl)-1,2,3,6-tétrahydro-2,6-dioxo-9H-purine-8-yl)cinnamique ;
ester d'éther méthylique de nonaéthylèneglycol d'acide (E)-4-((1,3-bis(2-fluorobenzyl)-1,2,3,6-tétrahydro-2,6-dioxo-9H-purine-8-yl)cinnamique ;
ester d'éther méthylique de nonaéthylèneglycol d'acide (E)-4-((1,3-bisphénéthyl)-1,2,3,6-tétrahydro-2,6-dioxo-9H-purine-8-yl)cinnamique ;
ester d'éther méthylique de nonaéthylèneglycol d'acide (E)-4-((1-cyclohexylméthyl-3-méthyl)-1,2,3,6-tétrahydro-2,6-dioxo-9H-purine-8-yl)cinnamique ;
ester d'éther méthylique de nonaéthylèneglycol d'acide (E)-4-((1-H-3-(2-méthyl-propyl)-1,2,3,6-tétrahydro-2,6-dioxo-9H-purine-8-yl)cinnamique ;
ester d'éther méthylique de nonaéthylèneglycol d'acide (E)-4-(1,3-bis(4-fluorobenzyl)-1,2,3,6-tétrahydro-2,6-dioxo-9H-purine-8-yl)cinnamique ;
ester d'éther dodécylique d'hexaéthylèneglycol d'acide (E)-4-(1,3-bis(cyclohexylméthyl)-1,2,3,6-tétrahydro-2,6-dioxo-9H-purine-8-yl)cinnamique ;
ester d'éther méthylique de nonaéthylèneglycol d'acide (E)-4-(1,3-bis(cyclobutylméthyl)-1,2,3,6-tétrahydro-2,6-dioxo-9H-purine-8-yl)cinnamique ;
ester d'éther méthylique de nonaéthylèneglycol d'acide (E)-4-(1-méthyl-3-cyclohexylméthyl)-1,2,3,6-tétrahydro-2,6-dioxo-9H-purine-8-yl)cinnamique ;
ester d'éther méthylique de nonaéthylèneglycol d'acide (E)-4-(1-méthyl-3-isobutyl)-1,2,3,6-tétrahydro-2,6-dioxo-9H-purine-8-yl)cinnamique ;
ester d'éther méthylique de nonaéthylèneglycol d'acide (4-(1,3-bis(cyclohexylméthyl)-1,2,3,6-tétrahydro-2,6-dioxo-9H-purine-8-yl)benzoïque ;
ester d'éther méthylique de nonaéthylèneglycol d'acide (E)-4-(1,3-bis(cyclohexyl)-1,2,3,6-tétrahydro-2,6-dioxo-9H-purine-8-yl)cinnamique ;
nonaéthylèneglycol-méthyl-éther-amide d'acide (E)-4-(1,3-bis(cyclohexylméthyl)-1,2,3,6-tétrahydro-2,6-dioxo-9H-purine-8-yl)cinnamique ;
nonaéthylèneglycol-méthyl-éther-amide d'acide (E)-4-(1,3-bis(cyclopentylméthyl)-1,2,3,6-tétrahydro-6-oxo-2-thioxo-9H-purine-8-yl)cinnamique ;
nonaéthylèneglycol-méthyl-éther-amide d'acide (E)-4-(1,3-bis(-2-méthyl-propyl)-1,2,3,6-tétrahydro-2,6-dioxo-9H-purine-8-yl)cinnamique ;
nonaéthylèneglycol-méthyl-éther-amide d'acide (E)-4-((1-cyclohexylméthyl-3-propyl)-1,2,3,6-tétrahydro-2,6-dioxo-9H-purine-8-yl)cinnamique ;
nonaéthylèneglycol-méthyl-éther-amide d'acide 4-(1,3-bis(cyclohexylméthyl)-1,2,3,6-tétrahydro-2,6-dioxo-9H-purine-8-yl)benzoïque ;
N-méthyl-nonaéthylèneglycol-méthyl-éther-amide d'acide 4-(1,3-bis(cyclohexylméthyl)-1,2,3,6-tétrahydro-2,6-dioxo-9H-purine-8-yl)benzoïque ;
ester d'éther méthylique de nonaéthylèneglycol d'acide (E)-4-(1,3-bis(cyclohexylméthyl)-2,3,6,7-tétrahydro-2,6-dioxo-7-benzyl-1H-purine-8-yl)cinnamique ;
ester d'éther méthylique de nonaéthylèneglycol d'acide (E)-4-(1,3-bis(cyclohexylméthyl)-2,3,6,7-tétrahydro-2,6-dioxo-7-(2-oxo-2-phényléthyl)-1H-purine-8-yl)cinnamique ;
ester d'éther méthylique de nonaéthylèneglycol d'acide (E)-4-(1,3-bis(cyclohexylméthyl)-2,3,6,7-tétrahydro-2,6-dioxo-7-méthyl-1H-purine-8-yl)cinnamique ;
ester d'éther méthylique de nonaéthylèneglycol d'acide (E)-4-(1,3-bis(cyclohexylméthyl)-2,3,6,7-tétrahydro-2,6-dioxo-7-(2-propynyl)-1H-purine-8-yl)cinnamique ;
ester d'éther méthylique de nonaéthylèneglycol d'acide (E)-4-(1,3-bis(cyclohexylméthyl)-2,3,6,7-tétrahydro-2,6-dioxo-7-(2-oxo-2-méthyléthyl)-1H-purine-8-yl)cinnamique ;
ester d'éther méthylique de nonaéthylèneglycol d'acide (E)-4-(1,3-bis(cyclohexylméthyl)-2,3,6,7-tétrahydro-2,6-dioxo-7-(3-morpholinopropyl)-1H-purine-8-yl)cinnamique ;
ester d'éther méthylique de nonaéthylèneglycol d'acide (E)-4-(1,3-bis(cyclohexylmethyl)-2,3,6,7-tétrahydro-2,6-dioxo-7-éthyl-1H-purine-8-yl)cinnamique ;
ester d'éther méthylique de nonaéthylèneglycol d'acide (E)-4-(1,3-bis(cyclohexylméthyl)-2,3,6,7-tétrahydro-2,6-dioxo-7-(2-éthoxy-2-oxoéthyl)-1H-purine-8-yl)cinnamique ;
ester d'éther méthylique de nonaéthylèneglycol d'acide (E)-4-(1,3-bis(cyclohexylméthyl)-2,3,6,7-tétrahydro-2,6-dioxo-7-(2-méthyl-2-propényl)-1H-purine-8-yl)cinnamique ;
ester d'éther méthylique de nonaéthylèneglycol d'acide (E)-4-(1,3-bis(cyclohexylméthyl)-2,3,6,7-tétrahydro-2,6-dioxo-7-(cyanométhyl)-1H-purine-8-yl)cinnamique ;
ester d'éther méthylique de nonaéthylèneglycol d'acide 4-(1,3-bis(cyclohexylméthyl)-2,3,6,7-tétrahydro-2,6-dioxo-7-benzyl-1H-purine-8-yl)benzoïque ;
ester d'éther méthylique de nonaéthylèneglycol d'acide 4-(1,3-bis(cyclohexylméthyl)-2,3,6,7-tétrahydro-2,6-dioxo-7-méthyl-1H-purine-8-yl)benzoïque ;
ester d'éther méthylique de nonaéthylèneglycol d'acide 4-[(1,3-bis(cyclohexylméthyl)-2,3,6,7-tétrahydro-2,6-dioxo-7-méthyl-1H-purine-8-yl)phényl]propionique ;
nonaéthylèneglycol-méthyl-éther-amide d'acide (E)-4-(1,3-bis(cyclohexylméthyl)-2,3,6,7-tétrahydro-2,6-dioxo-7-méthyl-1H-purine-8-yl)cinnamique ;
nonaéthylèneglycol-méthyl-éther-amide d'acide (E)-4-(1,3-bis(cyclohexylméthyl)-2,3;6,7-tétrahydro-2,6-dioxo-7-benzyl-1H-purine-8-yl)cinnamique ;
nonaéthylèneglycol-méthyl-éther-amide d'acide 4-(1,3-bis(cyclohexylméthyl)-2,3,6,7-tétrahydro-2,6-dioxo-7-benzyl-1H-purine-8-yl)benzoïque ;
nonaéthylèneglycol-méthyl-éther-amide d'acide 4-(1,3-bis(cyclohexylméthyl)-2,3,6,7-tétrahydro-2,6-dioxo-7-méthyl-1H-purine-8-yl)benzoïque ;
1,3-bis-(cyclohexylméthyl)-8-[4-(2,5,8,11,14,17,20,23,26,29-décaoxatriacont-1-yl)phényl]-3,7-dihydro-1H-purine-2,6-dione ;
ester d'éther méthylique de nonaéthylèneglycol d'acide (E)-3-[5-[1,3-bis(cyclohexylméthyl)-2,3,6,7-tétrahydro-2,6-dioxo-1H-purine-8-yl]-2-thiényl]-2-propénoïque;
nonaéthylènéglycol-méthyl-éther-amide d'acide 6-(1,3-bis(cyclohexylméthyl)-1,2,3,6-tétrahydro-2,6-dioxo-9H-purine-8-yl)nicotinique ;
N-cyclopropylméthyl-nonaéthylèneglycol-méthyl-éther-amide d'acide (E)-3-(1,3-bis(cyclohexylméthyl)-1,2,3,6-tétrahydro-2,6-dioxo-9H-purine-8-yl)cinnamique ;
hexaéthylèneglycol-benzyl-éther-amide d'acide (E)-4-(1,3-bis(cyclohexylméthyl)-1,2,3,6-tétrahydro-2,6-dioxo-9H-purine-8-yl)cinnamique ;
ester d'éther méthylique d'heptaéthylèneglycol d'acide (E)-4-[(3-cyclohexylméthyl)-2,3,6,7-tétrahydro-2,6-dioxo-7-méthyl-1H-purine-8-yl]cinnamique ;
ester d'éther méthylique de nonaéthylèneglycol d'acide (E)-4-[(3-cyclohexylméthyl)-2,3,6,7-tétrahydro-2,6-dioxo-7-méthyl-1H-purine-8-yl]cinnamique ;
ester d'éther méthylique de nonaéthylèneglycol d'acide (E)-4-[(3-cyclohexylméthyl)-2,3,6,7-tétrahydro-2,6-dioxo-1,7-diméthyl-1H-purine-8-yl]cinnamique ;
éther méthylique d'heptaéthylèneglycol de 4-[1,3-bis-(cyclohexylméthyl)-1,2,3,6-tétrahydro-2,6-dioxo-9H-purine-8-yl]benzylamine ;
chlorhydrate d'éther méthylique de N-heptaéthylèneglycol de 4-[1,3-bis(cyclohexylméthyl)-1,2,3,6-tétrahydro-2,6-dioxo-9H-purine-8-yl]benzylamine ;
éther méthylique de N-nonaéthylèneglycol de 4-[1,3-bis(cyclohexylméthyl)-1,2,3,6-tétrahydro-2,6-dioxo-9H-purine-8-yl]benzylamine ;
1,3-bis(cyclohexylméthyl)-8-[3-(2,5,8,11,14,17,20,23,26,29-décaoxatriacont-1-yl)phényl]-3,7-dihydro-1H-purine-2,6-dione ;
ester d'éther méthylique d'heptaéthylèneglycol d'acide (E)-4-(1,3-bis(cyclohexylméthyl)-2,3,6,7-tétrahydro-2,6-dioxo-7-méthyl-1H-purine-8-yl)cinnamique ;
ester d'éther méthylique de pentaéthylèneglycol d'acide (E)-4-(1,3(cyclohexylméthyl)-2,3,6,7-tétrahydro-2,6-dioxo-7-méthyl-1H-purine-8-yl)cinnamique ;
ester d'éther méthylique de nonaéthylèneglycol d'acide (E)-4-(1,3-bis(cyclohexylméthyl)-2,3,6,7-tétrahydro-2,6-dioxo-7-propyl-1H-purine-8-yl)cinnamique ;
ester d'éther méthylique de décaéthylèrteglycol d'acide (E)-4-(1,3-bis(cyclohexylméthyl)-1,2,3,6-tétrahydro-2,6-dioxo-9H-purine-8-yl)cinnamique ;
ester d'éther méthylique de nonaéthylèneglycol d'acide (E)-4-(1,3-bis(cyclohexylméthyl)-1,2,3,6-tétrahydro-2,6-dioxo-9H-purine-8-yl)cinnamique ;
ester d'éther méthylique de nonaéthylèneglycol d'acide (E)-3-[1,3-bis(cyclohexylméthyl)-1,2,3,6-tétrahydro-2,6-dioxo-9H-purine-8-yl]cinnamique ;
nonaéthylèneglycol-méthyl-éther-amide d'acide (E)-4-[1,3-bis(cyclohexylméthyl)-1,2,3,6-tétrahydro-2,6-dioxo-9H-purine-8-yl)cinnamique ; et
ester d'éther méthylique de nonaéthylèneglycol d'acide (E)-4-[1,3-bis(cyclohexylméthyl)-1,2,3,6-tétrahydro-2,6-dioxo-9H-purine-8-yl]benzoique ; et
leurs produits de solvatation pharmaceutiquement acceptables.

4. Utilisation suivant la revendication 1 ou 2, dans laquelle le composé de formule (I) est l'ester d'éther méthylique de nonaéthylèneglycol d'acide (E)-4-(1,3-bis-(cyclohexylméthyl)-1,2,3,6-tétrahydro-2,6-dioxo-9H-purine-9-yl)cinnamique ou un de ses produits de solvatation pharmaceutiquement acceptables.
